(19) Europäisches Patentamt
European Patent Office
Office européen des brevets

(11) EP 2 864 279 B1

(12) # EUROPEAN PATENT SPECIFICATION

(45) Date of publication and mention of the grant of the patent:
**02.11.2016 Bulletin 2016/44**

(21) Application number: **13724281.4**

(22) Date of filing: **23.05.2013**

(51) Int Cl.:
***C07C 35/32*** (2006.01) ***A61K 31/045*** (2006.01)
***A61P 1/00*** (2006.01) ***A61P 29/00*** (2006.01)

(86) International application number:
**PCT/EP2013/060614**

(87) International publication number:
**WO 2013/174917 (28.11.2013 Gazette 2013/48)**

(54) **COMPOUNDS FOR USE IN THE TREATMENT OF AUTOIMMUNE INFLAMMATORY DISEASE**

VERBINDUNGEN ZUR VERWENDUNG BEI DER BEHANDLUNG VON ENTZÜNDLICHEN AUTOIMMUNKRANKHEITEN

COMPOSÉS DESTINÉS À ÊTRE UTILISÉS DANS LE TRAITEMENT D'UNE MALADIE AUTO-IMMUNE INFLAMMATOIRE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **24.05.2012 US 201261651167 P**

(43) Date of publication of application:
**29.04.2015 Bulletin 2015/18**

(73) Proprietor: **Venantius Limited**
**Dublin 2 (IE)**

(72) Inventors:
- **FRANKISH, Neil**
  **Dublin 14 (IE)**
- **SHERIDAN, Helen**
  **Dublin 14 (IE)**

(74) Representative: **O'Brien, John Augustine et al**
**John A. O'Brien & Associates**
**Third Floor,**
**Duncairn House,**
**14 Carysfort Avenue**
**Blackrock, Co Dublin (IE)**

(56) References cited:
**WO-A1-97/20802** **DE-A1- 19 619 036**
**US-A1- 2010 152 220**

- **BIOORGANIC & MEDICINAL CHEMISTRY LETTERS, vol. 19, no. 20, 2009, pages 5927-5930, XP002702871, cited in the application**
- **E. PASSANTE ET AL: "In vitro inhibition of rat basophilic leukaemia mast cell (RBL-2H3) degranulation by novel indane compounds", INFLAMM. RES. SUPPLEMENT I, vol. 57, 2008, pages S15-S16, XP002702872, cited in the application**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 864 279 B1

## Description

Background

In WO97/20802A we describe a series of dimeric indane molecules with four different chemical scaffolds. Within this group of molecules we elaborated 2, 2 coupled dimers. The synthesis and biological activity for several 2-alkylated derivatives of this scaffold are presented as is their biological activity. The chemical scaffolds of the indane dimers disclosed in WO97/20802A are quite diverse. Amongst the many compounds disclosed two specific diastereoisomeric molecules [*(R, S and S, R)* 2-Benzyl-2, 3-dihydro-1H, 1'H-2, 2'-biinden-1-ol and (*S, S and R, R)* 2-Benzyl-2, 3-dihydro-1H, 1'H-2, 2'-biinden-1-ol] are referred to. Biological data for these diastereoisomers led to the conclusion in WO97/20802A that *R,S and S, R-* 2-Benzyl-2, 3-dihydro-1H, 1'H-2, 2'-biinden-1-ol had the potential to treat allergic conditions.

Inflamm. Res., 57 (2008) p. 15,discloses that *(R, S and S, R)* 2-Benzyl-2, 3-dihydro-1H, 1'H-2, 2'-biinden-1-ol and (*S, S and R, R)* 2-Benzyl-2, 3-dihydro-1H, 1'H-2, 2'-biinden-1-ol demonstrate dose dependent inhibition of β-hexosaminidase release from RBL-2H3 cells with greater biological activity residing in diastereoisomer *(R, S and S, R)* 2-Benzyl-2, 3-dihydro-1H, 1'H-2, 2'-biinden-1-ol, on the basis that the marginal effect did attain statistical significance. It is observed in this paper that taken together the data indicated that the diastereoisomer (*S, S and RR*) 2-Benzyl-2, 3-dihydro-1H, 1'H-2, 2'-biinden-1-ol may be a good candidate for the treatment of histamine related diseases. However, in a subsequent study reported in Bioorganic & Medicinal Chemistry Letters, 19(20), 2009, Pg 5927-5930; the effects of the two compounds *(R, S and SR*)-2-Benzyl-2, 3-dihydro-1H, 1'H-2, 2'-biinden-1-ol and (*S, S and R, R)* 2-Benzyl-2, 3-dihydro-1H, 1'H-2, 2'-biinden-1-ol on β-hexosaminidase release from RBL-2H3 cells stimulated by calcium ionophore A23187 (as a measure of mast cell stabilisation) was recorded. Both compounds caused a dose-dependant inhibition of enzyme release. Experiments on inhibition of arachidonic acid-induced mouse ear swelling were performed on *(R, S and S, R)* 2-benzyl-2, 3-dihydro-1H, 1'H-2, 2'-biinden-1-ol and (*S, S and R, R)* 2-benzyl-2, 3-dihydro-1H, 1'H-2, 2'-biinden-1-ol No difference in the activity of the two diastereoisomers on mouse ear swelling was observed. Compounds (*R, S and S, R)* 2-benzyl-2, 3-dihydro-1H, 1'H-2, 2'-biinden-1-ol and (*S, S and R, R)* 2-benzyl-2, 3-dihydro-1H, 1'H-2, 2'-biinden-1-ol (3C9) were equipotent in all systems studied. These data suggested the potential to influence eicosanoid mediated inflammatory processes as observed in acute inflammation and analgsia.

This invention relates to new compounds for use in the treatment of inflammatory bowel disease and T cell mediated immune inflammatory diseases.

Inflammatory bowel disease (IBD) consists of two idiopathic inflammatory diseases ulcerative colitis and Crohn's disease (CD) [Gastroenterology 2011;140:1785-1794]. The greatest distinction between ulcerative colitis and Crohn's disease is the range of inflamed bowel tissue. Inflammation in Crohn's disease is discontinuously segmented known as regional enteritis, while ulcerative colitis is superficial inflammation extending proximally and continuously from the rectum. At present the exact cause of Crohn's disease is unknown. The disease seems to be related to an exaggerated mucosal immune response to infection of the intestinal epithelium because of an imbalance of pro-inflammatory and immune-regulatory molecules. The inheritance patterns of Crohn's disease suggest a complex genetic component of pathogenesis that may consist of several combined genetic mutations. Currently no specific diagnostic test exists for Crohn's disease, but as understanding of pathogenesis is improved so will the testing methods. Treatment of Crohn's disease consists of inducing remission by anti-inflammatories followed by general immune-suppressants. Emergent therapeutic options focus on specific inflammatory pathways which will halt inflammation and induce remission in patients with Crohn's disease. Although several anti-inflammatory and immunosuppressive agents have been used to treat Crohn's disease, the two FDA-approved therapies for Crohn's disease are Remicade, a TNFα-antagonist marketed by Johnson & Johnson, and Entocort, a coated, corticosteroid capsule marketed by AstraZeneca.

Ulcerative colitis is more prevalent than Crohn's disease and also has an uncertain aetiology. A family history of inflammatory bowel disease is the most important independent risk factor, but environmental factors are important, with a higher incidence in developed countries. It has a bimodal pattern of incidence with the main onset peak between ages 15 and 30 years, and a second smaller peak between ages 50 and 70 years. Episodes of previous gastrointestinal infection double the risk of subsequent development of ulcerative colitis, which suggesting that acute intestinal infection might lead to changes in gut flora, hence triggering the start of a chronic inflammatory process in genetically predisposed individuals. Maintenance of remission of the disease is by treatment with 5-aminosalicylic acid in a variety of formulations, with active disease being treated by corticosteroids or by anti-TNF-α antibodies such as Remicade.

IBD is not to be confused with Inflammatory Bowel Syndrome (IBS) which does not cause inflammation. Unlike ulcerative colitis patients, IBS sufferers show no sign of disease or abnormalities when the colon is examined.

Statements of Invention

In particular, this invention provides a compound of the specific stereochemical Formula 1:

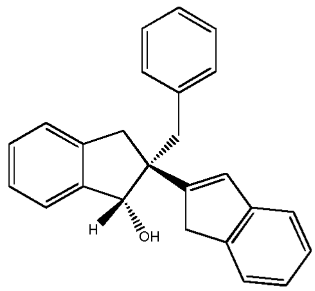

Formula 1

Also provided are pharmacologically acceptable salts of the compound of Formula (I).

The absolute stereochemistry of the enantiomer represented in Formula I has been established by single crystal X-ray analysis.

The invention further provides for a pharmaceutical composition comprising of the compound described above.

The active compound may be present in the medicament for use in man at a single dose to achieve the desired effect. For example the final dose may be between 0.1 and 10 mg/Kg.

It may be possible to administer the compound of the invention in the form of a bulk active chemical. It may however, be preferred that the compound be administered in the form of a pharmaceutical formulation or composition. Such formulations may comprise of one or more pharmaceutically acceptable excipients, carrier of diluents.

The compound in the invention may be administered in different ways. The compound may be administered orally. Preferred pharmaceutical formulations for oral administration include tablets, capsules, solutions, suspensions of syrups.

The pharmaceutical formulations may be provided in a form for modified release such as time release capsule or tablet.

The medicament may be administered orally, parenterally, intranasally, transcutaneously or by inhalation.

The compound may be applied topically or as suppositories.

The compound of the invention and salts thereof are useful in prophylaxis and/or treatment of inflammatory bowel disease or other inflammatory autoimmune diseases with similar aetiology involving T-cell proliferation or function. Such diseases include rheumatoid arthritis, psoriasis, psoriatic arthritis, multiple sclerosis, eosinophilic fasciitis, demyelinating neuropathies, and autoimmune vasculitis (including Behcet's disease).

The compound is useful for the prophlaxis or treatment of a disease mediated by IL2.

The compound is useful for the prophlaxis or treatment of inflammatory bowel disease.

The compound is useful for the prophlaxis or treatment of Crohn's disease.

The compound is useful for the prophlaxis or treatment of ulcerative colitis.

Brief description of the drawings

The invention will be more clearly understood from the following description thereof given by way of example only, in which:-

**Fig. 1** is an X-ray crystal structure showing relative stereochemistry for the diastereoisomer **6.**

**Fig. 2** is an X-ray crystal structure showing the absolute stereochemistry of the *S, S*-enantiomer (confirming structural Formula **1)** corresponding to 4-bromobenzoic acid ester derivative **(13)** of compound **10;**

**Fig. 3 is** an X-ray powder diffraction pattern (XPRD) of compound **13:** (a) experimental XPRD pattern for compound **13;** (b) the simulated XPRD pattern calculated from single-crystal structure of compound **13;**

**Fig. 4** is a bar chart of the effect of indomethacin and compounds **5** & **6** (300(μg/ear in acetone) on arachidonic acid mouse ear swelling (results are expressed as a mean ± SEM, n >=4);

**Fig. 5** is a bar chart of the effect of Ciclosporin (CicA) (50mg/kg) and compounds **5** & **6** (10 mg/kg) on mBSA-stimulated mouse paw swelling (results are expressed as a mean ± SEM, n >= 12);

**Fig. 6** is a bar chart of the effect of Ciclosporin (CicA) (50mg/kg) and compounds **5** & **6** (10 mg/kg) on SRBC-stimulated mouse paw swelling (results are expressed as a mean ± SEM, n >= 5);

**Fig. 7** is a bar chart of the effect of Ciclosporin (CicA) (15mg/kg), dexamethasone (15mg/kg) and compounds **5** & **6** (15 mg/kg) on oxazolone-stimulated mouse ear swelling (ear punch weight) (results are expressed as a mean ± SEM, n = 6);

**Fig. 8** is a graph of the effect of vehicle and compounds **6, 9** & **10** (30mg/kg) (p.o.) on body weight in 5 % DSS colitis (values expressed as a mean ± SEM, n=6 (non-DSS n=4));

**Fig. 9** is a graph of the effect of vehicle and compounds **6, 9** & **10** (30mg/kg) (p.o.) on disease activity index (DAI) in 5 % DSS colitis on day 7 (values expressed as a mean ± SEM, n=6); and

**Fig 10** is a bar chart of the effect of vehicle and compounds **6, 9 & 10** (30mg/kg) (p.o.) on disease activity index (DAI) in 5 % DSS colitis on day 7 (values expressed as a mean ± SEM, n=6).

Detailed Description of the Invention

General Chemical Background

Ketone (compound **1)** which has a chiral centre at C-2 is a racemic mixture of a pair of enantiomers, named compound **2** and compound **3.** This enantiomeric mix is not separated.

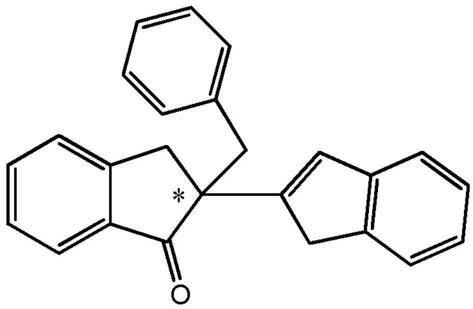

**Compound 1**

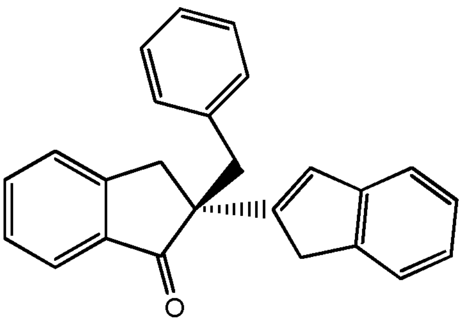

(*R*)-2-benzyl-2,3-dihydro-1*H*,1'*H*-[2,2'-biinden]-1-one

**Compound 2**

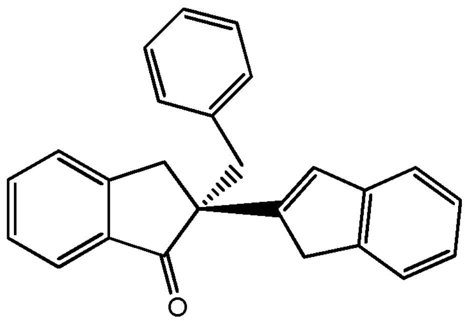

(*S*)-2-benzyl-2,3-dihydro-1*H*,1'*H*-[2,2'-biinden]-1-one

**Compound 3**

Enantiomers of compound 1

Reduction of compound **1** with chiral reduction agents for example $LiAlH_4$, or the like results in the formation of compound **4** which is a mixture of the two diastereoisomers each composed of two enantiomers.

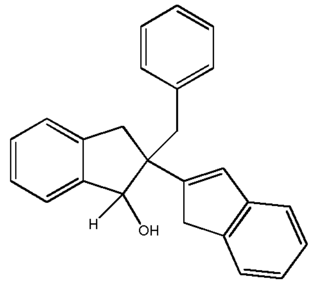

L

Compound **4**

The diastereosisomers which compose compound **4** have the (*RSISR*) or (*RRISS*) configuration. These diastereoisomers can be separated chromatographically into 5 and 6, in varying ratios depending on reaction conditions

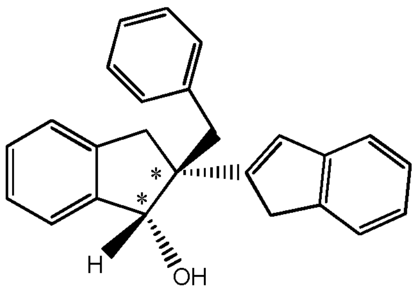

Diastereoisomer **5** (*RS/SR* configuration)

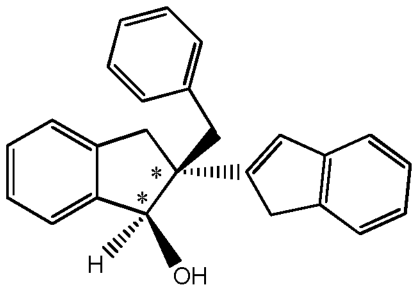

Diastereoisomer **6** (*RR/SS* configuration)

The relative stereochemistry from single crystal X-Ray analysis of Diastereoisomer **6** is given in Fig. 1.
The relative stereochemistry has been assigned by single crystal X-Ray analysis of Diastereoisomer 6 as elaborated in Bioorganic & Medicinal Chemistry Letters 2009, 15;19(20):5927-30.
Each diastereoisomer is a mixture of two enantiomers. Diastereoisomer **5** is a mixture of two separate enantiomers 7 and **8**

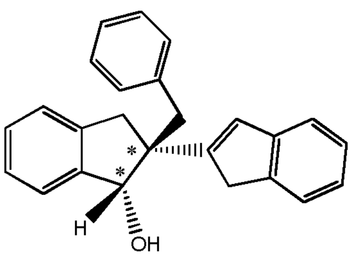

**7/8**

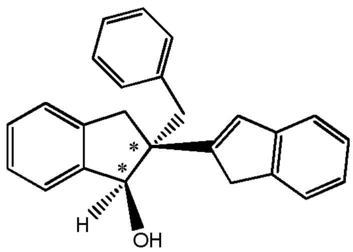

**7/8**

The relative stereochemistry **of 7** and **8** has been assigned by reference to the single crystal X-ray data for **6.** The absolute stereochemistry has not been assigned.
Diastereoisomer **6** is a mixture of two separate enantiomers **9** and **10**

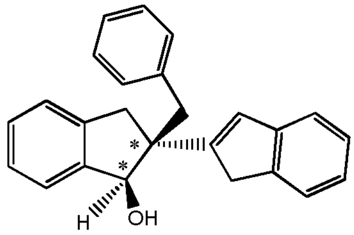

9

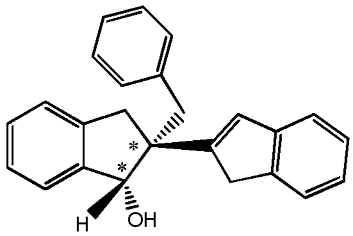

10

The absolute stereochemistry of **10** has been assigned by single crystal X-Ray analysis of its 4-bromobenzoic acid ester derivative **13** (Figure 2).

General Reaction Procedures

General synthetic procedures for the coupling of enantiomeric mixtures as exemplified for diastereoisomer **6** laid out in WO97/20802A.

One of ordinary skill in the art would recognise variations in synthetic schemes which are appropriate for the preparation of compounds of the current invention.

Reduction of alkylated coupled product and separation of diastereoisomers **5** and **6**

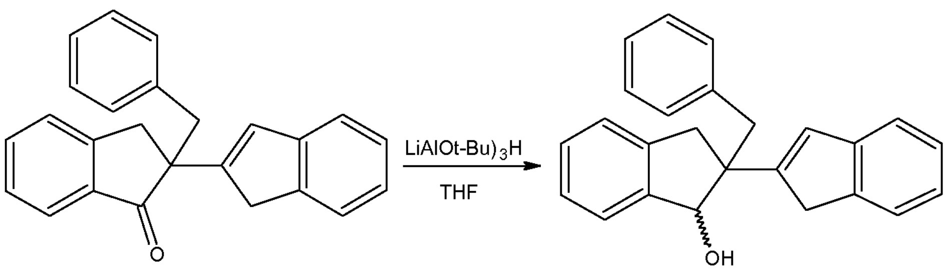


To a stirred solution of the alkylated product (4.64 g, 14 m.mol) in THF (115 mL) was added lithium tri-*tert*-butoxyaluminohydride (11.57 g, 45 mmol) in one portion. The solution was stirred at room temperature and monitored by TLC (90 : 10 hexane : ethyl acetate). After 1 h the reaction was quenched by pouring onto an ice-water mixture. The THF was removed *in vacuo* and the aqueous solution was stirred with an equivalent amount of ethyl acetate for 15 min. The resulting mixture was poured into a separatory funnel and allowed to separate, and the procedure was repeated with the aqueous layer. The combined organic extracts were washed with water and brine, dried over $MgSO_4$ and evaporated to give the product (4.0 g, 85%) as a pale yellow oil which contains a 50 : 50 mixture of the two diastereoisomers **5** and **6.**

The two diastereoisomers **5** and **6** were separated by flash chromatography as described below (see 1.4). For TLC, solvent system is 80: 20 hexane : ethyl acetate and the TLC plate must be 8-9 cm in order to see both isomers clearly. Column size: 30 cm silica gel x 3 cm column.

The crude product is first dissolved in $CH_2Cl_2$ and adsorbed onto silica gel. The column is packed with 100% hexane and loaded with the adsorbed silica gel. Further hexane (~300 mL) is used to push the adsorbed silica gel onto the column proper.

500 mL of 100 : 0.5 hexane : ethyl acetate, 500 mL of 100 : 1, 500 mL of 100 : 2500 mL of 100 : 4, 500 mL of 100 : 5, 500 mL of 100 : 6, 500 mL of 100 : 7 (at the end of this fraction, the product usually starts to appear) 500 mL of 100 : 8 to end.

The maximum amount loaded onto the column using this method was 6 g. In all cases, generally 50 : 50 of the two diastereoisomers was recovered. Generally, a mixed fraction is also recovered.

**Analytical Results for 5 and 6**

Diastereoisomer **5:** White solid, m.p. 128-130 °C. $\delta_H$(300 MHz, $CDCl_3$): 3.00 (2H, dd, J 13.8 Hz, $CH_2$) [AB, centred at 3.00], 3.08 (2H, dd, J 15.8 Hz, $CH_2$) [AB system], 3.41 (2H, dd, J 22.9 Hz, $CH_2$) [AB system], 5.02 (1H, m,

C**H**OH), 6.64 (1H, s, C**H**=C), 6.91-6.94 (2H, m, ArH), 7.10-7.31 (9H, br m, ArH), 7.35-7.40 (2H, m, ArH). $\delta_C$ (75.5 MHz, $CDC_3$):38.5 [$CH_2$], 40.5 [$CH_2$], 43.4 [$CH_2$], 56.4 [Cq], 81.9, 120.6, 123.5, 124.4, 125.0, 126.3, 127.0, 128.0, 128.7, 130.3, 130.4, 138.1 [Cq], 141.7 [Cq], 143.4 [C.q], 143.8 [Cq], 144.5 [Cq], 151.1 [Cq].

Diastereoisomer **6:** Clear oil or clear crystals m.p. 123-124°C. $\delta_H$(300 MHz, $CDCl_3$): 2.51 (1H, d, J 5.3 Hz, OH), 2.94 (2H, dd, J 13.4 Hz, $CH_2$) [AB system], 2.98, (2H, dd, J 15.6 Hz, $CH_2$) [AB system], 3.46 (2H, dd, J 22.5 Hz, $CH_2$) [AB system], 5.17 (1H, d, J 5.1 Hz, C**H**OH), 6.44 (1H, s, C**H**=C), 6.82-6.85 (2H, m, ArH), 7.09-7.14 (4H, m, ArH), 7.22-7.26 (5H, m, ArH), 7.36-7.39 (2H, m, ArH). $\delta_C$ (75.5 MHz, $CDCl_3$): 38.3 [$CH_2$], 38.4 [$CH_2$], 39.9 [$CH_2$], 55.8 [$CH_2$], 82.9, 120.4, 123.4, 123.9, 124.0, 124.7, 126.0, 126.2, 126.8, 127.7, 128.1, 128.3, 130.1, 138.3 [Cq], 140.6 [Cq], 142.9 [Cq], 143.9 [Cq], 144.7 [Cq], 153.2 [Cq].

X-Ray Crystal analysis of **6**

The relative stereochemistry of the diastereoisomer diastereoisomer C being a mixture of two enantiomers was determined by single crystal X-ray analysis of a crystal formed from tert Butyl Methyl Ether as shown in Figure 1.

Separation of constituent enantiomers of diastereoisomer **5** and **6**

Method: the single diastereoisomer **5** or **6** was derivatised with N-BOC D-phenylalanine; the subsequently formed diastereoisomers were separated then the phenylalanine group was removed by hydrolysis.

Diastereoisomers **5** and **6**

Preparation of N-BOC D-phenylalanine derivative of **5** or **6**.

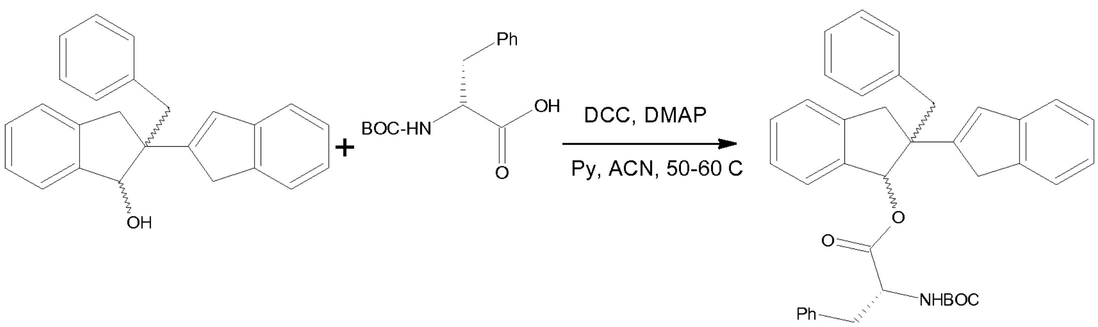


To a stirred solution of **5** or **6** (1.2 mmol, 0.406 g) and N-BOC D-phenylalanine (1.5 mmol, 0.398 g) in $CH_3CN$ (8 mL) was added pyridine (1.5 mmol, 0.12 mL). The mixture was stirred at room temperature and a solution of DCC (1.5 mmol, 0.309 g) and DMAP (10mol%, 0.015 g) in $CH_3CN$ (2 mL) was added dropwise. After a short time, a white precipitate was seen. The reaction was heated to 50-60 °C for 16h. (In the case of **6,** the reaction took approximately 2h; for **5,** overnight stirring was required). On cooling, the reaction mixture was filtered and the dicyclohexylurea precipitate washed well with $CH_3CN$. The $CH_3CN$ was evaporated *in vacuo* and the residue taken up in ethyl acetate (20 mL). The ethyl acetate extract was washed with 1N $H_2SO_4$ (20 mL), sat. $NaHCO_3$ (20 mL) and brine (20 mL), dried over $MgSO_4$ and evaporated to give a yellow oil which contained two diastereoisomers by TLC (80 : 20 hexane : MTBE). The diastereoisomers were separated by chromatography using 95 : 5 hexane : MTBE as eluent to give diastereoisomer **11:** 0.335g (48%); diastereoisomer **12** 0.323 g (46%).

Hydrolysis of N-BOC D-phenylalanine derivatives **(11** and **12)** of Diastereoisomer **5** or **6** to give enantiomers: **7** and **8** (from **5) 9** and **10** (from **6)**

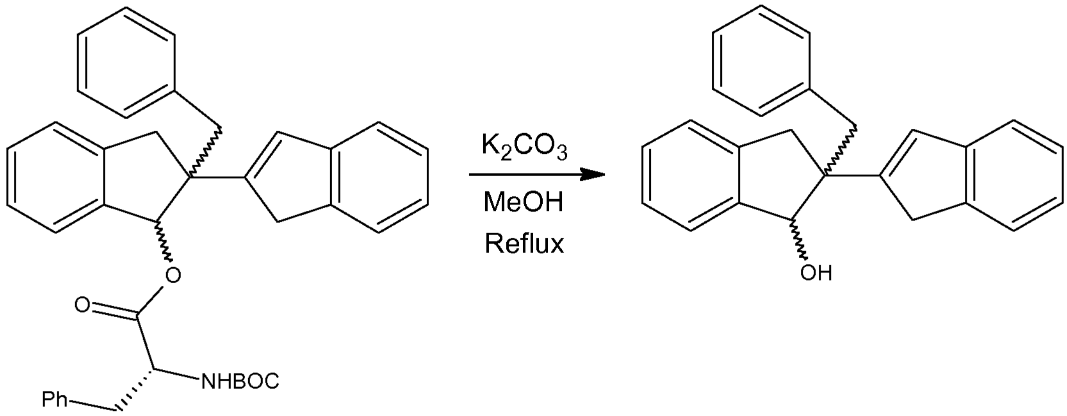


**11** or **12** **5** or **6**

To a stirred solution of N-BOC D-phenylalanine derivative **11** or **12** of Diastereoisomer **5** or **6** (1.12 mmol, 0.66 g) in methanol (25 mL) was added potassium carbonate (1.23 mmol, 0.17 g). The reaction mixture was heated at reflux and monitored by TLC (80 : 20 hexane : MTBE). After 2h, no further starting material was seen.The methanol was removed *in vacuo* and the solid residue taken up in water and ethyl acetate. The layers were separated and the aqueous layer extracted with ethyl acetate (2 x 25 mL). The combined organic layers were washed with water (3 x 50 mL), brine (50 mL), dried over $MgSO_4$ and evaporated. The crude product was chromatographed using 90 : 10 hexane : MTBE as eluent to give 0.34 g (90%) of the product as a white solid.

**Analytical results of separated enantiomers of 5 and 6.**

A) Enantiomer No.1 from Diastereoisomer **5:** Enantiomer **7**

Enantiomer 7: Off-white solid, m.p. 97-99°C. $[\alpha]_D$:-1.764 (5.61%, $CHCl_3$). $\delta_H$ (300 MHz, $CDCl_3$):1.71 (1H, d, J 8.0 Hz, OH), 3.05 (2H, dd, J 13.8 Hz, $CH_2$) [AB system], 3.08 (2H, dd, J 15.8 Hz, $CH_2$) [AB system], 3.43 (2H, dd, J 22.7 Hz, $CH_2$) [AB system], 5.05 (1H, d, J 7.9 Hz, C**H**OH), 6.67 (1H, s, C**H**=C), 6.91-6.97 (2H, m, ArH), 7.12-7.19 (4H, m, ArH), 7.22-7.33 (5H, m, ArH), 7.37-7.42 (2H, m, ArH). $\delta_C$ (75.5 MHz, $CDC_3$):38.4 [$CH_2$], 40.4 [$CH_2$], 43.4 [$CH_2$], 56.4 [Cq], 81.8, 120.6, 123.5, 124.3, 124.9, 125.0, 126.3, 127.0, 128.0, 128.7, 130.2, 130.4, 138.0 [Cq], 141.7 [$C_q$], 143.3 [$C_q$], 143.7 [$C_q$], 144.5 [$C_q$], 151.0 [$C_q$]

Enantiomer No.2 Diastereoisomer 5: Enantiomer 8

Enantiomer **8**: Off-white solid. M.p. 115-119°C, $[\alpha]_D$: +1.458 (8.98%, $CHCl_3$). $\delta_H$ (300 MHz, $CDCl_3$): 1.75 (1H, d, J 7.9 Hz, OH), 2.95 (2H, dd, J 13.8 Hz, $CH_2$) [AB system], 3.20 (2H, dd, J 15.6 Hz, $CH_2$) [AB system], 3.42 (2H, dd, J 22.7 Hz, $CH_2$) [AB system], 5.04 (1H, d, J 7.7 Hz, C**H**OH), 6.66 (1H, s, C**H**=C), 6.92-6.95 (2H, m, ArH), 7.11-7.32 (9H, br m, ArH), 7.36-7.41 (2H, m, ArH). $\delta_C$ (75.5 MHz, $CDCl_3$): 38.4 [$CH_2$], 40.4 [$CH_2$], 43.4 [$CH_2$], 56.4 [Cq], 81.8, 120.6, 123.5, 124.3, 124.9, 125.0, 126.3, 127.0, 128.0, 128.7, 130.2, 130.4, 138.0 [Cq], 141.6 [Cq], 143.3 [Cq], 143.7 [Cq], 144.5 [Cq], 151.0 [Cq]

Enantiomer No.1 from Diastereoisomer **6**: Enantiomer **9**

Enantiomer **9**: Pale yellow Off-white solid. M.p. 57-60°C. $[\alpha]_D$: +64.72 (7.79%, $CHCl_3$) + 100 (1.20%, MeOH). $\delta_H$ (300 MHz, $CDCl_3$): 2.08 (1H, d, J 8.0 Hz), 2.97 (2H, dd, J 13.5 Hz, $CH_2$) [AB system], 3.01 (2H, dd, J 15.6 Hz, $CH_2$) [AB system], 5.21 (1H, d, J 8.0 Hz, C**H**OH), 6.47 (1H, s, C**H**=C), 6.85-6.87 (2H, m, ArH), 7.12-7.16 (4H, m, ArH), 7.21-7.29 (5H, m, ArH), 7.39-7.42 (2H, m, ArH).

Enantiomer No.2 from Diastereoisomer **6:** Enantiomer **10**

Enantiomer **10:** Pale yellow Off-white solid, m.p. 59-64°C. $[\alpha]_D$: 65.52 (7.67%, $CHCl_3$), - 90.10 (1.08%, MeOH)). $\delta_H$ (300 MHz, $CDCl_3$):2.09 (1H, d, J 7.5 Hz), 2.96 (2H, dd, J 13.4 Hz, $CH_2$) [AB system], 3.01 (2H, dd, J 15.6 Hz, $CH_2$) [AB system], 3.49 (2H, dd, J 22.5 Hz, $CH_2$) [AB system], 5.21 (1H, d, J 7.0 Hz, C**H**OH), 6.47 (1H, s, C**H**=C), 6.84-6.89 (2H, m, ArH), 7.01-7.19 (4H, m, ArH), 7.21-7.32 (5H, m, ArH), 7.38-7.42 (2H, m, ArH).

Synthesis of (1S, 2S)-2-benzyl-2,3-dihydro-2-(1H-inden-2-yl)-1H-inden-1-yl 4-bromobenzoate **13.**

To a stirred solution of (*1S*,*2S*)-2-benzyl-2,3-dihydro-2-(1H-inden-2-yl)-1H-inden-1-ol (0.06 g, 0.177 mmol) and 4-bromobenzoic acid (0.05 g, 0.266 mmol) in dry dichloromethane (5 mL) was added 4-dimethylaminopyridine (0.03 g, 0.266 mmol), diisopropylethylamine (0.05 mL, 0.266 mmol) and 2, 6-dichlorobenzoyl chloride (0.04 mL, 0.266 mmol) under an atmosphere of nitrogen. After an hour the reaction was quenched by the addition of saturated sodium bicarbonate solution (10 mL) and the product was extracted with diethyl ether (3 x 20 mL). The organic layers were combined and dried over anhydrous magnesium sulphate and concentrated *in vacuo.* The resulting oily residue was then purified by flash column chromatography (stationary phase; silica gel 230-400 mesh, mobile phase; hexane:ethyl acetate, 10:1) to yield target product, (*1S*,*2S*)-2-benzyl-2,3-dihydro-2-(1H-inden-2-yl)-1H-inden-1-yl 4-bromobenzoate, as a pale yellow oil (0.08 g, 99%). Pale crystals from acetonitrile and isopropanol (v:v, 1:1), m.p.. 156-158 °C. HRMS (+$Na^+$): 543.0930 m/z; required 543.0912; m/z; $C_{32}H_{25}O_2BrNa$. $\delta_H$(400 MHz, $CDCl_3$): 3.17 (1H, *d*, J=13.56Hz, C$\underline{\mathbf{H}}_2$), 3.19 (1H, *d*, J=15.42 Hz, C$\underline{\mathbf{H}}_2$), 3.27 (1H, *d*, J=22.52 Hz, C$\underline{\mathbf{H}}_2$), 3.366 (1H*, d,* J=13.56 Hz, C$\underline{H}_2$), 3.371 (1H, *d*, J=15.42 Hz, C$\underline{H}_2$), 3.41 (1H*, d,* J=22.52 Hz, C$\underline{H}_2$), 6.57 (1H, *s*, C$\underline{H}$=C), 6.64 (1H, s, C$\underline{H}$OC=O), 6.94-6.95 (2H, *overlapping signals,* Ar-$\underline{H}$), 7.15 (1H, *dt,* $J_1$=1.23 Hz, $J_2$=7.29 Hz, Ar-$\underline{H}$), 7.15-7.19 (3H, *m*, Ar-$\underline{H}$), 7.21-7.24 (2H, *m*, Ar-$\underline{H}$), 7.27 (1H, *d,* J=7.16 Hz, Ar-$\underline{H}$), 7.31-7.32 (2H, *m*, Ar-$\underline{H}$), 7.36 (1H, *overlapping d*, J=7.60 Hz, Ar-$\underline{H}$), 7.39 (1H, *overlapping d*, J=7.42 Hz, Ar-$\underline{H}$), 7.67 (2H, *overlapping d*, J=8.68 Hz, Ar-$\underline{H}$), 8.05 (2H, *overlapping d*, J=8.52 Hz, Ar-$\underline{H}$).. $\delta_C$(100 MHz, $CDCl_3$): 39.6 ($\underline{C}H_2$), 40.4 ($\underline{C}H_2$), 40.8 ($\underline{C}H_2$), 54.9 (quat. $\underline{C}$), 83.8 ($\underline{C}$HOC=O), 120.6 (tert. $\underline{C}$), 123.5 (tert. $\underline{C}$), 124.3 (tert. $\underline{C}$), 124.6 (tert. $\underline{C}$), 125.7 (tert. $\underline{C}$), 126.31 (tert. $\underline{C}$), 126.33 (tert. $\underline{C}$), 126.9 (tert. $\underline{C}$), 2 x 127.9 (2 x tert. $\underline{C}$), 128.5 (quat. $\underline{C}$), 129.08 (tert. $\underline{C}$), 129.1 (quat. $\underline{C}$), 129.5 ($\underline{C}$H=C), 2 x 130.0 (2 x tert. $\underline{C}$), 2 x 131.3 (2 x tert. $\underline{C}$), 2 x 131.9 (2 x tert. $\underline{C}$), 137.9 (quat. $\underline{C}$), 140.4 (quat. $\underline{C}$), 142.2 (quat. $\underline{C}$), 142.7 (quat. $\underline{C}$), 144.4 (quat. $\underline{C}$), 151.6 (CH=$\underline{C}$), 165.6 ($\underline{C}$=OOAr).

HPLC Method

Column: Hypersil BDS C18, Wavelength: 220 nm, Flow rate: 1mL/min. Mobile phase: 80: 20 $CH_3CN$ : 0.1% aq. acetic acid. Sample: 1mg/mL made up in $CH_3CN$.

Using this system, diastereoisomers **5** and **6** do not separate but have a retention time of 6.9 min.

Chiral HPLC separation of Enantiomers of Diastereoisomer 6

Preparative Method: Column 250 x 20mm CHIRALPAK ADH 5μm. Mobile Phase 50/50/0.1 Methanol / Ethanol / Diethylamine. Flow rate: 14ml/min. Detection: UV 230nm, Temperature: 25°C

**Analytical Method:** Column 250 x 4.6mm CHIRALPAK ADH 5μm. Mobile Phase 50/50/0.1 Methanol / Ethanol / Diethylamine. Flow rate: 0.7ml/min. Detection: UV 230nm, Temperature: 25°C.

Method : Chiral Separation of Enantiomers of Diastereoisomer **6** (**9** and **10**).

**Analytical Method:** Column 250 x 20mm CHIRALPAK®IA 5μm. Mobile Phase Heptane/EtOH 95:5. Flow rate: 1ml/min. Detection: DAD 230nm, Temperature: 25°C

| | **First Eluting enantiomer 9** | **Second Eluting enantiomer 10** |
|---|---|---|
| **Retention time** | 11.7 min | 13.8 min |
| $\alpha_{[D]}$ | 64.72 (7.79%, $CHCl_3$) | -65.52 (7.67%, $CHCl_3$) |

X-Ray Studies

A single crystal X-ray analysis (Figure 2) was carried out on the 4-bromobenzoic acid ester (compound **13**) derivative of compound **10,** using a Bruker Apex DUO Diffractometer and the parameters outlined in Table 1.

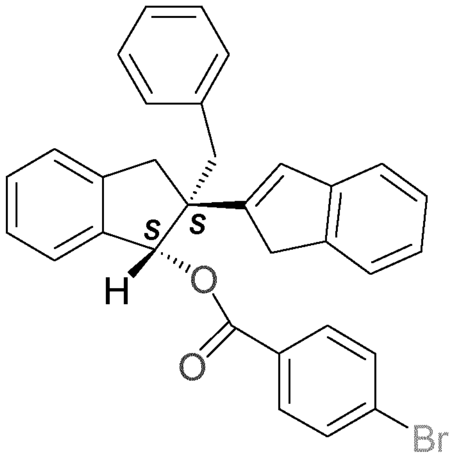

**Table 1. Data collection and structure refinement for compound 13; 4-bromobenzoic acid ester of compound 10.**

| | |
|---|---|
| Diffractometer | Bruker Apex DUO |
| Radiation source | Microfocus (Cu) X-ray Source, Cu $K\alpha$ |
| Data collection method | Omega and Phi scans |
| Theta range for data collection | 5.14 to 59.96° |
| Index ranges | -12<=h<=9, -6<=k<=5, -8<=1<=16 |
| Reflections collected | 2049 |
| Independent reflections | 1693 [R(int) = 0.0488] |
| Coverage of independent reflections | 62.8 % |
| Variation in check reflections | N/A |
| Absorption correction | Multi-scan |
| Max. and min. transmission | 1.00000 and 0.384 |
| Structure solution technique | direct methods |
| Structure solution program | SHELXS-97 (Sheldrick, 2008) |
| Refinement technique | Full-matrix least-squares on $F^2$ |
| Refinement program | SHELXL-97 (Sheldrick, 2008) |
| Function minimized | $\Sigma\ w(F_o^2 - F_c^2)^2$ |
| Data / restraints / parameters | 1693 / 99 / 317 |
| Goodness-of-fit on $F^2$ | 1.025 |
| Final R indices | 1607 data; $I>2\sigma(I)$ R1 = 0.0884, wR2 = 0.2159 |
| | all data R1 = 0.0900, wR2 = 0.2186 |
| Weighting scheme | $w=1 / [\sigma^2 (F_o^2)+(0.1997P)^2+0.0000P]$ |
| where $P=(F_o^2+2F_c^2)/3$ | |
| Absolute structure parameter | 0.0(1) |
| Extinction coefficient | 0.0000(9) |
| Largest diff. peak and hole | 1.298 and -2.002 eÅ$^{-3}$ |
| R.M.S. deviation from mean | 0.177 eÅ$^{-3}$ |

**Refinement summary:**

| | |
|---|---|
| Ordered Non-H atoms, XYZ | freely refining |
| Ordered Non-H atoms, U | Anisotropic |

(continued)

| | |
|---|---|
| H atoms (on carbon), XYZ | Idealized positions riding on attached atoms |
| H atoms (on carbon), U | Appropriate multiple of U(eq) for bonded atom |
| H atoms (on heteroatoms), XYZ | freely refining |
| H atoms (on heteroatoms), U | Isotropic |

The structure of compound **13** was solved and refined using the Bruker SHELXTL Software Package, using the space group P 1 21 1, with Z = 1 for the formula unit, $C_{32}H_{25}Br_1O_2$, with one molecule of compound **13** in the asymmetric unit (Table 2).

**Table 2. Sample and crystal data for compound 13**

| | | |
|---|---|---|
| Crystallization solvents | Acetonitrile and Isopropanol | |
| Crystallization method | Slow evaporation | |
| Empirical formula | $C_{32}H_{25}Br_1O_2$ | |
| Formula weight | 521.43 | |
| Temperature | 146(2) K | |
| Wavelength | 1.54178 Å | |
| Crystal size | 0.10 x 0.10 x 0.10 mm Crystal habit Colourless Plate Crystal system Monoclinic | |
| Space group | P 1 21 1 | |
| Unit cell dimensions | *a* = 13.668(3) Å | α = 90° |
| *b* = 6.0697(8) Å | | β= 105.655(17)° |
| *c* = 14.8879(3) Å | γ = 90° Volume 1188.6(4) $A^3$ | |
| Z | 1 | |
| Density (calculated) | 0.728 g/$m^3$ | |
| Absorption coefficient | 1.287 $mm^{-1}$ | |
| F(000) | 268 | |

The absolute stereochemistry was determined as *S, S* at COOJ and COOR for compound 13. The assignment was made from consideration of both the Flack parameter which was determined to be 0.0(1) and from the *a priori* knowledge of the stereochemistry of the ester former.

The theoretical x-ray powder diffraction pattern from the single crystal X-ray structure was in agreement with the experimental powder diffraction pattern (Figure 3), which confirmed the stereochemistry shown in Figure 2.

Effect of Compounds in Models of Inflammation

The biological activity of the compounds was assessed in a variety of acute inflammatory and immune inflammatory models.

Methodology

Mouse Ear Oedema

The arrachidonic mouse ear oedema model is an acute inflammation model based on activation of ecosanoid pathways. The mouse ear oedema model was performed using BalbC mice (25-35g), of either sex. The ear was treated by the topical application (10μl) of solvent (acetone 100%) or test drug (300μg/ear in acetone) or Indomethacin (300μg/ear in acetone). After one hour, oedema was induced by the topical application of arachidonic acid (10μl at 0.4g/ml in acetone). The width of each ear was measured, both before and 60min after the induction of oedema, using a micrometer screw gauge. Ear oedema was calculated by comparing the ear width before and after induction of oedema and expressed as percentage normal. Animals were killed using $CO_2$, without recovering from sedation.

Delayed-type hypersensitivity (DTH) Methods

Delayed-type hypersensitivity (DTH) is a cell-mediated immune response which is protective against intracellular

bacteria, fungi and some viruses. However when inappropriately deployed, it can cause extensive tissue damage in auto-immune inflammatory diseases such as rheumatoid arthritis and allograft rejection. Th1 cells have been proposed to be the inducer of this type of hypersensitivity since the resulting IFNγ production would activate macrophages However in some cases, DTH has been induced by a Th2 response. Models of DTH are often used to assess T cell mediated immune responses *in vivo.*

Cytokines involved in this process include IL-2, IL-3, GM-CSF, TNFα and IFNγ. Anti-IL-2 receptor antibodies administered during the sensitisation phase have been shown to suppress DTH. IL-12, produced from the antigen presenting cells (APCs) is also significant in cell-mediated immunity through the induction of IFNγ from NK cells and T cells and also enhances the cytotoxic activity ofNK cells.

Studies were undertaken in three different DTH models: the Methylated Bovine Serum Albumin (mBSA) model, the Sheep Red Blood Cell (SRBC) Model and the Oxazolone Contact Hypersensitivity (CHS) model.

<u>Methylated Bovine Serum Albumin (mBSA) model</u>

CD-1 mice (25-35gms) were anaesthetised with halothane and immunised intradermal (i.d.) with mBSA/Freunds complete adjuvant containing *Mycobacterium butyricum* (FCA(B)) emulsion at four sites (62.Sμg/25μl at each site) on the shaved chest on day 1. The mBSA/FCA(B) is prepared by emulsifying equal volumes of mBSA and FCA(B) solutions (the mBSA solution was first prepared in sterile isotonic saline at a concentration of 5mg/mL). On days 8 and 9, mice were dosed intraperitoneal (i.p.) with either 1% carboxymethylcellulose (CMC), CycA or a novel compound in 1% CMC. Two hours after the second i.p. dose (day 9), anaesthetised mice were challenged, by injecting mBSA in saline subcutaneous (s.c.) in the dorsal surface of the right hind paw and s.c. in the left hind paw with saline alone (20μl each injection). 24 hours later, mice were sacrificed by cervical dislocation, and the swelling of each paw was measured in triplicate with a plethysmometer.

$$\text{Paw swelling (\% difference)} = \frac{\{\text{mBSA injected paw volume (mls)}\} - \{\text{saline injected paw volume (mls)}\}}{\text{Saline injected paw volume (mls)}} \times 100\%$$

<u>Sheep Red Blood Cell (SRBC) Model</u>

The sheep red blood cells were first cleaned and counted as follows. 5mls of the SRBC were spun down in a centrifuge at 3000rpm for 10 minutes. The supernatant was removed and phosphate buffered saline (PBS), pH 7.4 added to the pellet. The pellet was mixed with the PBS buffer (5mls) and spun down in a centrifuge at 3000rpm for 10 minutes. The pellet was washed twice more with PBS buffer as described above. The pellet was then reconstituted in 5mls of PBS buffer following the final wash. 10μl of a 1 in 200 dilution of the sample of reconstituted sheep red blood cells (in trypan blue stain) was used to fill the counting chamber of a haemacytometer and the number of sheep red blood cells counted.

Male CD-1 mice (30-40gms) were sensitised on day zero. The negative control group was sensitised with 100μl PBS intraperitoneally (i.p.), while the positive control group and drug treated groups were sensitised with $1x10^7$ SRBC (100μl i.p.).

Animals were challenged 5 days later with SRBC. The SRBC were cleaned and counted as described above. Animals anaesthetised with halothane were challenged s.c. by injecting SRBC ($3x10^8$ cells in 20μl) into the right hind paws and PBS (20μl) into the left hind paws. This was done for all groups including the negative control group.

Drug treated groups were dosed i.p on day 4 and 2 hours prior to challenge on day 5. The negative control and positive control groups were dosed with 1% CMC using the same dosing schedule.

The difference in paw swelling between the saline-injected and SRBC-challenged contralateral paw of each mouse was calculated by measuring paw volume (mls) using a plethysmometer. This was carried out 24 hours after challenge to SRBC with triplicate measurements made on each paw. This was done as described previously for the mBSA delayed-type hypersensitivity model

Oxazolone Contact Hypersensitivity (CHS) model

Female Balb/C mice (30-40gms) were sensitised on day 0 with 20µl of 2% oxazolone in acetone on each ear (10µl on the inner and outer aspects of both ears). All mice were challenged on day 5 with 20µl of 2% oxazolone in acetone only on the right ear; again, 10µl on both the inner and outer aspects of the ear. Immediately following this, the mice were treated with test compound. The test compound was administered in exactly the same way as the oxazolone, with 10µl on each side of the right ear. All compounds were prepared in acetone at a concentration of 15mg/ml; 300µg/ear. For the positive control group, acetone was administered. All animals were killed 24hrs later and the percentage increase in ear swelling was calculated. This was done in two ways. The thickness of the unchallenged left ear and the challenged right ear were measured using a micrometer caliper (µm). The increase in weight of both ears was also measured using a 5mm biopsy punch (mgs). The percentage increase in oedema was then calculated for both weight and thickness by expressing the difference between the unchallenged left ear and the challenged right ear as a percentage of the left ear control.

Statistics

Comparisons between groups of data was performed by 1-way ANOVA using Graphpad Prism software. Results are displayed as a mean ± SEM. Where indicated, statistical significance is indicated by asterisks according to the following criteria:

| P value | Wording | Summary |
|---|---|---|
| < 0.0001 | Extremely significant | *** |
| 0.0001 to 0.001 | Extremely significant | *** |
| 0.001 to 0.01 | Very significant | ** |
| 0.01 to 0.05 | Significant | * |
| ≥ 0.05 | Not significant | ns |

Effect on Inflammatory Models

Arrachidonic mouse ear swelling.

Arrachidonic acid (10µl at 0.4g/ml in acetone) increased ear thickness of the right ear by 86% over the left ear. Indomethacin (300µg/ear in acetone) reduced ear swelling to 40%. In comparison, compounds **5** & **6** (300µg/ear in acetone) reduced ear swelling to 62% and 50% respectively (P<0.05). There was no significant difference in the degree of inhibition of ear swelling of the two compounds (P>0.05) See Fig. 4.

Conclusion: The diastereoisomers **5** and **6** are equipotent with the non-steroidal antiinflammatory drug, indomethacin. There is no statistical difference in terms of antiinflammatory effects of the two diastereosiomers **5** and **6** in this arachidonic acid mouse ear model of inflammation. These results would suggest the possibility of **5** and **6** having application in the treatment of inflammation and alleviation of pain.

Effect on methylated bovine serum albumin (mBSA) mouse paw swelling

On challenge with mBSA to the right paw in sensitised mice, paw volume increased by 107% over saline-injected left paws. Ciclosporin (50mg/kg) reduced paw swelling to 29% and compounds **5** & **6** (10 mg/kg i.p.) reduced right paw swelling to 72% and 71% respectively (Fig 5).

Conclusion: The mBSA stimulated mouse paw swelling model is a delayed type hupersensitivity model which exhibits inflammation by mechanisms outside the classical eicosonoid pathways suggested by the arachidonic acid mouse ear model. The mBSA results are in keeping with the observation of equipotency for **5** and **6** from the AA model. These mBSA results show no statistical difference in terms of antiinflammatory effects of the two diastereosiomers **5** and **6** which have moderate and statistically significant activity in this model. This suggests that **5** and **6** may have application in the treatment of autoimmune inflammatory disease.

Effect on sheep red blood cell (SRBC) - stimulated mouse paw swelling.

Injection of SRBCs into the right paws of unsensitised negative control mice increased paw volume by 24% whereas in sensitised animals, challenge by SRBCs increased right paw swelling to 89% in those animals administered vehicle only. Ciclosprorin (50mg/kg) reduced paw swelling to 57%. Compounds **5** & **6** (30 mg/kg) reduced paw swelling

to SRBC challenge to 60% and 52% respectively. There was no significant difference in the reduction in swelling by these two compounds (Fig 6).

Conclusion: The stimulated mouse paw swelling model (SRBC) is also a delayed type hupersensitivity model which exhibits inflammation by mechanisms outside the classical eicosonoid pathways. The SRBC results are in keeping with the observation of equipotency for **5** and **6** from the mBSA and AA models. These SRBC results show no statistical difference in terms of antiinflammatory effects of the two diastereosiomers **5** and **6** which have moderate and statistically significant activity in this model. This supports the observation from the mBSA study that **5** and **6** may have application in the treatment of autoimmune inflammatory disease.

Effect on Oxazolone Contact Hypersensitivity Mouse Ear Swelling

Administration of oxazolone to the right ears of oxazolone-sensitised mice increased ear punch weight by 107% in mice administered vehicle only. In contrast, ciclosporin (15 mg/kg) and dexamethasone (15 mg/kg) reduced the increase in ear punch weight to 46% and 42% respectively. However, neither compound **5** nor **6** had no significant effect on oxazolone mouse ear swelling ($P>0.05$) see Fig. 7.

Conclusion: The oxazolone-stimulated mouse ear swelling is a third delayed type hupersensitivity model which exhibits inflammation by mechanisms outside the classical eicosonoid pathways. Neither **5** nor **6** had activity in this model. In particular it was worth notinhg that there was no difference in activity between the two diastereoisomers **5** and **6** as previously observed with the mBSA, SRBC and AA models. The fact that diastereoisomers **5** and **6** had activity in two of the three DTH models suggests that they are showing specificity for processes involved in these models.

IL-2 secretion

Many of the immunosuppressive drugs used in the treatment of autoimmune diseases and organ transplant rejection, such as corticosteroids and immune suppressive drugs (ciclosporin, tacrolimus) work by inhibiting the production of IL-2 by antigen-activated T cells. Others (sirolimus) block IL-2R signalling, thereby preventing the clonal expansion and function of antigen-selected T cells [ref: Opposing functions of IL-2 and IL-7 in the regulation of immune responses Shoshana D. Katzman, Katrina K. Hoyer, Hans Dooms, Iris K. Gratz, Michael D. Rosenblum, Jonathan S. Paw, Sara H. Isakson, Abul K. Abbas. Cytokine 56 (2011) 116-121]. Cytokines can be produced by various cell populations and have been shown to augment or limit immune responses to pathogens and influence the autoimmune response. One family of cytokines, which uses the common receptor gamma chain (cc), a component of receptors for interleukin (IL)-2, IL-4, IL-7, IL-9, IL-15 and IL-21, has been classically defined as growth and survival factors. IL-2 production can induce an immune response by promoting the proliferation and generation of CD4+ Th1, CD4+ Th2 and CD8+ CTL effector cells.

In contrast IL-2 can inhibit the immune response by promoting the survival and functionality of natural (thymic) regulatory T-cells (Tregs), promoting the generation of induced (peripheral) Tregs and inhibiting the generation of CD4+ Th17 effector cells [ref: IL-2 and autoimmune disease. Cytokine & Growth Factor Reviews 13 (2002) 369-378]. Interleukin-2/IL-2R deficiency with time leads to multiorgan inflammation and the formation of autoantibodies of various specificities. Depending on the genetic background, death occurs within a few weeks to a few months, mostly from autoimmune hemolytic anemia or inflammatory bowel disease (IBD) [ref. Ulcerative colitis-like disease in mice with a disrupted interleukin-2 gene. Cell 1993;75:253-61].

IL-2 signalling has been shown to be important in both the initiation and regulation of immune responses. In these dual and opposing roles, IL-2 acts to balance immune response, both driving immune cell activation and subsequent reduction. The potential clinical applicability of either augmenting or inhibiting signals mediated by IL-2 is significant and includes cancer, autoimmune inflammatory diseases, organ transplantation and HIV.

Due to the presence of two chrial centres in each of the diastereoisoemrs **5** and **6,** and therefor the presence of two enantiomers in each diastereoisomer **5 (7** and **8)** and **6 (9** and **10)** we isolated the single enantiomers **7, 8, 9** and **10** and evaluated their effects on IL2 release from Jurkat cells, alongside their corresponding diastereoisomers.

Materials and Methods

Methodology

The T cell line Jurkat 6.1 (ATCC) was used. Cells were pre-treated for 30min with 1μM or 10μM of the respective compound and then stimulated with plate-bound anti-CD3 (BD Pharmingen) and anti-CD28 (AnCell). DMSO was used as vehicle control. The immunosuppressive agent Cyclosporine A was used as a control for inhibition of IL-2 production. After 24hs the supernatant was collected and IL-2 secretion was measured by ELISA.

Cell culture

Jurkat T cells were cultured in RMPI-1640 medium containing 10 % Foetal Bovine Serum, Penicillin/Streptomycin and L-Glutamine in T-75cm2 flasks at 37°C and 5 % CO2. Cells were seeded into new culture flasks at a density of 1-3 x 105 cells per ml and maintained at a density of 0.5 -1 x 106 cells per ml.

Activation of Jurkat T lymphocytes

(A) Anti-TCR / CD28-mediated activation of Jurkat cells: Experiments involving anti-TCR/CD28-mediated activation of Jurkat T lymphocytes were carried out on 24-well cell culture plates. The plates were coated with a 1/100 dilution of rabbit anti-mouse IgG in sterile PBS (1X) and incubated overnight at 4OC. Unbound antibody was aspirated and the wells were gently rinsed with warm sterile PBS. The PBS was aspirated and the wells were coated with a 1/100 dilution of anti-TCR antibody (OKT-3) and a 1/200 dilution of anti-CD28 antibody (both antibodies pre-mixed prior to addition to the plates) in sterile PBS. The plates were then incubated for 1-2 hours at 37°C. Unbound antibody was aspirated and the wells were gently rinsed with warm PBS. The PBS was left on the plate until the Jurkat cells were ready to be added to the plate. The Jurkat cells were harvested from the T-75cm2 cell culture flasks and resuspended at a density of 0.5 x 106 cells per ml. The compounds were dissolved at a specified stock concentration (see results section) and diluted to the required concentration by adding the drug to the cell suspension. The cells were then pre-incubated with the compounds or with an equivalent volume of drug vehicle control (DMSO) for 30 minutes prior to adding the cell suspension to the antibody-coated plates. Cyclosporine A (1 [μM) was used as a positive control to inhibit IL-2 production from activated T cells and was preincubated with the 6 cells as described above in parallel. The cells were then incubated on the plates with the acti-TCR/CD28 antibodies for 24 hours at 37°C and 5% CO2 in a humidified atmosphere. After 24 hours, the cell suspension was removed from the plates and the cells were pelleted by centrifugation. The cell culture supernatant was saved and analysed for secreted IL-2 by ELISA. The cell pellet was saved and cell viability was measured using Acridine Orange/Ethidium Bromide cell viability staining solution.

(B) PHA / PMA-mediated activation of Jurkat cells Jurkat cells were resuspended at a concentration of 0.5 x 106 cells per ml and the Pharmatrin compounds at the desired concentration or an equivalent volume of drug vehicle control (DMSO) were added to the cells and incubated for 30 minutes. Cyclosporine A (1 ocM) was used as a positive control to inhibit IL-2 production from activated T cells and was preincubated with the cells as described above in parallel. PHA and PMA were added to the cell suspension (10 ocg/ml and 10 ng/ml respectively) and the cells were plated onto uncoated 24-well plates. The cells were incubated on the plates for 24 hours at 37°C and 5 % $CO_2$ in a humidified atmosphere. After 24 hours, the cell suspension was removed from the plates and the cells were pelleted by centrifugation. The cell culture supernatant was saved and analysed for secreted IL-2 by ELISA.

Measurement of cell viability

The cell pellet was resuspended in 20 μl of Acridine Orange / Ethidium Bromide staining solution. A portion of this cell suspension was placed on a haemocytometer and analysed under a fluorescence microscope. Viable cells appeared green whilst non-viable cells had a characteristic orange appearance. The ratio of viable cells: non-viable cells were calculated for each activation time point / drug treatment.

ELISA assay

The cell culture supernatants were analysed for IL-2 by ELISA (96-well plate format) using the Human IL-2 ELISA kit from R&D Systems Europe. This assay was carried out using the standard protocol outlined by the company. Samples and standards (recombinant human IL-2 provided by R&D Systems) were analysed in triplicate. The colour formed in each well (proportional to the amount of IL-2 in the samples or standards) was read using a 96-well plate reader (Titertek Multiscan, Medical Supply Company) at 450nm. Analysis of data and chart generation was constructed using the Microsoft Excel program.

**Diastereoisomer6**

| Sample | []pg/ml (1) | []pg/ml (2) | []pg/ml (3) | % Control (1) | % Control (2) | % Control (3) |
|---|---|---|---|---|---|---|
| R | 13.60 | -24.95 | 1.51 | 18.62 | -38.21 | 1.15 |

(continued)

| Sample | []pg/ml (1) | []pg/ml (2) | []pg/ml (3) | % Control (1) | % Control (2) | % Control (3) |
|---|---|---|---|---|---|---|
| DMSO 0.1% | 73.03 | 65.30 | 131.56 | 100.00 | 100.00 | 100.00 |
| Cyc. A | -0.25 | -18.73 | 2.56 | -0.34 | -28.68 | 1.94 |
| 60.3uM | 63.72 | 74.50 | 101.75 | 87.26 | 114.09 | 77.34 |
| 61uM | 58.72 | 51.60 | 112.44 | 80.41 | 79.02 | 85.47 |
| 6 3uM | 47.83 | 29.15 | 104.44 | 65.50 | 44.64 | 79.39 |
| 6 10uM | 27.72 | 36.15 | 75.19 | 37.96 | 55.36 | 57.15 |
| 6 30uM | 4.72 | 47.60 | 16.47 | 6.47 | 72.89 | 12.52 |
| 6 100uM | -10.11 | -19.75 | 1.97 | -13.85 | -30.25 | 1.50 |

**Enantiomer 9**

| Sample | []pg/ml (1) | []pg/ml (2) | []pg/ml (3) | % Control (1) | % Control (2) | % Control (3) |
|---|---|---|---|---|---|---|
| R | 13.60 | -24.95 | 1.51 | 18.62 | -38.21 | 1.15 |
| DMSO 0.1% | 73.03 | 65.30 | 131.56 | 100.00 | 100.00 | 100.00 |
| Cyc. A | -0.25 | -18.73 | 2.56 | -0.34 | -28.68 | 1.94 |
| 9 0.3uM | 111.72 | 73.45 | 139.63 | 152.99 | 112.48 | 106.13 |
| 9 1uM | 120.56 | 65.45 | 114.13 | 165.09 | 100.23 | 86.75 |
| 9 3uM | 78.78 | 81.75 | 53.47 | 107.88 | 59.11 | 40.64 |
| 9 10uM | 37.72 | 85.60 | 36.16 | 51.66 | 131.09 | 27.48 |
| 9 30uM | -4.56 | 16.35 | -3.88 | -6.24 | 25.04 | -2.95 |
| 9 100uM | -3.72 | -5.75 | -5.28 | -5.10 | -8.81 | -4.01 |

**Enantiomer 10**

| Sample | []pg/ml (1) | []pg/ml (2) | []pg/ml (3) | % Control (1) | % Control (2) | % Control (3) |
|---|---|---|---|---|---|---|
| R | 13.60 | -24.95 | 1.51 | 18.62 | -38.21 | 1.15 |
| DMSO 0.1% | 73.03 | 65.30 | 131.56 | 100.00 | 100.00 | 100.00 |
| Cyc. A | -0.25 | -18.73 | 2.56 | -0.34 | -28.68 | 1.94 |
| 10 0.3uM | 79.17 | 88.15 | 58.63 | 108.41 | 134.99 | 44.56 |
| 10 1um | 64.17 | 57.50 | 104.44 | 87.87 | 88.06 | 79.39 |
| 10 3uM | 71.39 | 54.15 | 100.50 | 97.76 | 82.92 | 76.39 |
| 10 01uM | 42.50 | 45.60 | 80.25 | 58.20 | 69.83 | 61.00 |
| 10 30uM | 14.17 | 56.70 | 46.06 | 19.40 | 86.83 | 35.01 |
| 10 100uM | -11.33 | -6.30 | -0.78 | -15.52 | -9.65 | -0.59 |

**Diastereoisomer5**

| Sample | []pg/ml (1) | []pg/ml (2) | []pg/ml (3) | % Control (1) | % Control (2) | % Control (3) |
|---|---|---|---|---|---|---|
| R | -11.38 | -24.95 | 1.51 | -55.76 | -38.21 | 1.15 |
| DMSO 0.1% | 20.40 | 65.30 | 131.56 | 100.00 | 100.00 | 100.00 |
| Cyc. A | -10.48 | -18.73 | 2.56 | -51.35 | -28.68 | 1.94 |
| 5 0.3uM | 32.00 | 87.25 | 164.41 | 156.86 | 133.61 | 124.97 |
| 5 1uM | 21.85 | 64.65 | 127.84 | 107.11 | 99.00 | 97.18 |
| 5 3uM | 10.90 | 83.25 | 97.38 | 53.43 | 60.20 | 74.02 |
| 5 10uM | 8.75 | 63.80 | 70.72 | 42.89 | 97.70 | 53.76 |
| 5 30uM | 16.25 | 5.75 | 6.69 | 79.66 | 8.81 | 5.08 |
| 5 100uM | -10.40 | -13.05 | 0.28 | -50.98 | -19.98 | 0.21 |

**Enantiomer 7**

| Sample | []pg/ml (1) | []pg/ml (2) | []pg/ml (3) | % Control (1) | % Control (2) | % Control (3) |
|---|---|---|---|---|---|---|
| R | -11.38 | -24.95 | 1.51 | -55.76 | -38.21 | 1.15 |
| DMSO 0.1% | 20.40 | 65.30 | 131.56 | 100.00 | 100.00 | 100.00 |
| Cyc. A | -10.48 | -18.73 | 2.56 | -51.35 | -28.68 | 1.94 |
| 7 0.3uM | 44.65 | 93.65 | 112.94 | 218.87 | 143.42 | 85.85 |
| 7 1uM | 49.20 | 71.40 | 109.59 | 241.18 | 109.34 | 83.31 |
| 7 3uM | 43.25 | 45.10 | 97.25 | 212.01 | 69.07 | 73.92 |
| 7 10uM | 24.75 | 36.75 | 81.84 | 121.32 | 56.28 | 62.21 |
| 7 30uM | 10.10 | 27.80 | 32.00 | 49.51 | 42.57 | 24.32 |
| 7 100uM | 1.15 | -19.30 | -3.13 | 5.64 | -29.56 | -2.38 |

**Enantiomer 8**

| Sample | []pg/ml (1) | []pg/ml (2) | []pg/ml (3) | % Control (1) | % Control (2) | % Control (3) |
|---|---|---|---|---|---|---|
| R | -11.38 | -11.38 | 56.81 | -55.76 | -55.76 | 41.08 |
| DMSO 0.1% | 20.40 | 20.40 | 138.29 | 100.00 | 100.00 | 100.00 |
| Cyc. A | -10.48 | -10.48 | 3.19 | -51.35 | -51.35 | 2.31 |
| 8 0.3uM | 44.20 | 50.10 | 107.03 | 216.67 | 245.59 | 77.39 |
| 8 1uM | 37.95 | 39.65 | 111.75 | 186.03 | 194.36 | 80.81 |
| 8 3uM | 34.45 | 36.00 | 102.92 | 168.87 | 176.47 | 74.42 |
| 8 10uM | 20.15 | 25.65 | 81.78 | 98.77 | 125.74 | 59.13 |
| 8 30uM | -2.80 | -3.00 | 76.25 | -13.73 | -14.71 | 55.14 |
| 8 100uM | -12.55 | -12.80 | 8.11 | -61.52 | -62.75 | 5.87 |

**Effect of compounds 5 and 8 and ciclosporin of the anti-TCR/CD28 or PHA/PMA-mediated IL2 secretion from Jurkat T Cells.** The effect of the indicated concentrations of compounds on inhibition of IL-2 secretion from Jurkat cells is indicated as a percentage value (relative to DMSO control). In some cases compounds did not inhibit IL-2 secretion (and in fact gave a negative percentage value in comparison to DMSO), which is indicated in the table as NE (No Effect).

Conclusion

Compounds **5, 7, 8** and **6** inhibited IL2 release from Jurkat cells without affecting cell viability.

Inflammatory Bowel Disease (IBD)

Inflammatory bowel disease (IBD) consists of two idiopathic inflammatory diseases ulceritive colitis and Crohn's disease (CD). The greatest distinction between ulcerative colitis and Crohn's disease is the range of inflamed bowel tissue. Inflammation in Crohn's disease is discontinuously segmented known as regional enteritis, while ulceritive colitis is superficial inflammation extending proximally and continuously from the rectum. At present the exact cause of Crohn's disese is unknown. The disease seems to be related to an exaggerated mucosal immune response to infection of the intestinal epithelium because of an imbalance of pro-inflammatory and immune-regulatory molecules. The inheritance patterns of Crohn's disease suggest a complex genetic component of pathogenesis that may consist of several combined genetic mutations. Currently no specific diagnostic test exists for Crohn's disease, but as understanding of pathogenesis is improved so will the testing methods. Treatment of Crohn's disease consists of inducing remission by anti-inflammatories followed by general immune-suppressants. Emergent therapeutic options focus on specific inflammatory pathways which will halt inflammation and induce remission in patients with Crohn's disease. Although several anti-inflammatory and immunosuppressive agents have been used to treat Crohn's disease, the two FDA-approved therapies for Crohn's disease are Remicade, a TNFα-antagonist marketed by Johnson & Johnson, and Entocort, a coated, corticosteroid capsule marketed by AstraZeneca.

Inflammatory Bowel Disease - Murine Colitis Model

Dextran sulfate sodium (DSS) induced colitis is an experimental model which exhibits many of the symptoms

observed in human Ulcerative Colitis such as diarrhoea, bloody faeces, mucosal ulceration, and shortening of the colon and weight loss. It is therefore often used as a model for studying the pathogenesis of UC and screening of drugs used in the treatment of UC. An acute model (5% DSS) of colitis was used. This dose induces severe acute colitis, by day 6-7 mice will have rectal bleeding and will die by day 10-12.

Mice

Specific Pathogen-Free female Balb/c mice, 6-8 weeks of age, were obtained from a commercial supplier (Harlan, UK). Mice were fed irradiated diet and housed in individually ventilated cages (Tecniplast, UK) under positive pressure.

Dextran Sodium Sulphate (DSS) treatment.

Dextran sodium sulfate (DSS: 5%) was dissolved in the drinking water. Compounds were injected at a dose of 10 mg/kg i.p. on days 0-7, and mice were culled on day 8 or day 9, depending on the severity of the disease. The mice were checked each day for morbidity and weight of individual mice recorded. Induction of colitis was determined by weight loss, fecal blood, stool consistency, and, upon autopsy, length of colon and histology. Colons were recovered and stored at -20°C for immunological analysis. All compounds and experimental groups were randomly alphabetically labelled. Throughout experiments all data recording was performed in a blind manner. The codes on boxes/groups were not broken until after the data was analysed, i.e. boxes labelled A, B C etc were identified as untreated, DSS-treated, or DSS + compound treated.

To quantify the extent of colitis a disease activity index (DAI) was determined based on weight loss, occult blood and stool consistency. A score (Table 1) was given for each parameter, with the sum of the scores used as the DAI. For each treatment group n=6.

Administration of Compounds

All compounds were prepared for injection (0.1 mls intraperitoneal (i.p.) per 10g body weight) as a suspension in 0.5% carboxymethyl cellulose/2% Tween 80, at a dose of 10mg $kg^{-1}$. Compounds were initially dissolved in absolute ethanol and diluted 9+1with 0.5% carboxymethyl cellulose/2% Tween 80; this resulted in a fine precipitate in suspension.

Table 1.

| Score | Weight loss % | Stool consistency | Occult bleeding |
|---|---|---|---|
| 0 | None | normal | none |
| 1 | 1-3 | | |
| 2 | 3-6 | Loose stool[1] | Visible in stool |
| 3 | 6-9 | | |
| 4 | >9 | Diarrhea[2] | Gross bleeding[3] |

Definitions
[1]Loose stool - stool formed, but becomes a paste on handling.
[2]Diarrhoea - no stool formation fur stained around anus.
[3]Gross bleeding - fresh blood on fur around anus with extensive blood in the stool.

Results

DSS at 5% in the drinking water, induced a progressive weight loss compared to non-DSS mice and this weight loss was ameliorated by diastereoisomer **6** and by the *S, S* -enantiomer **10** but not by the *R, R*- enantiomer **9** (Fig. 8).

Of the pair of diastereoisomers **5** and **6,** only **6** caused a significant (P<0.01) inhibition of DIA in DSS-induced colitis (Figure 9). Of the two enantiomers of diastereoisomer **6, 5, 9** and **10,** only the *S, S* -enantiomer **10** caused a significant (P<0.0059) inhibition of DIA (Fig.9).

Compound **10** is the single active *S*, *S*-enantiomer, the value of the vehicle control being 6.833 ±1.276 being and that of the **10** treated group being 2.333±0.211 on DAI at day 7 (Figure 9).

Fig 10. Effect of vehicle and compounds **6, 9** & **10** (30mg/kg) (p.o.) on disease activity index (DAI) in 5 % DSS colitis on day 7. Values expressed as a mean ± SEM, n=6.

It is apparent, that there is a very significant and unexpected difference in activity between the diastereoisomers **5** and **6,** and most significantly between the single enantiomers **9** and **10.** We are observing very specific and selective activity for the enantiomer with the *S, S*-configuration. This activity shows that this molecule has the potential to treat immuno inflammatory conditions in the inflammatory bowel disease spectrum, including Crohn's disease and ulcerative colitis and that it operates by a different mechanism than the other enantiomers.

The invention is not limited to the embodiments described but may be varied in detail.

APPENDIX: 1

Abbreviations

| | |
|---|---|
| aq | aqueous |
| b.p. | boiling point |
| $CaCl_2$ | calcium chloride |
| $CDCl_3$ | chloroform-d |
| $CHCl_2$ | dichloromethane |
| dIW | distilled ionised water |
| DMSO | dimethyl sulphoxide |
| MTBE | Ether |
| EtOAc | ethyl acetate |
| EtOH | ethanol |
| $H_2O$ | water |
| $H_2SO_4$ | sulphuric acid |
| HCl | hydrochloric acid |
| IR | infra red |
| M | Molar |
| $MgCl_2$ | magnesium chloride |
| $MgSO_4$ | Magnesium sulphate |
| min | minutes |
| µl | microlitres |
| mM | milli-molar |
| m.p. | melting point |
| $N_aHCO_3$ | sodium hydrogen carbonate |
| $NaHCO_3$ | sodium bicarbonate |
| $NaH_2PO$ | sodium hydrogen phosphate |
| NaOH | sodium hydroxide |
| $Na_2SO_4$ | sodium sulphate |
| $NH_4Cl$ | ammonium chloride |
| NMR | nuclear magnetic resonance |
| RT | room temperature |
| S.E.M. | standard error of mean |
| THF | tetrahydrofuran |
| TLC | thin layer chromatography |
| µl | microliters |

APPENDIX 2

**5** (diastercomer) *(R,S and SR)* 2-Benzyl-2,3-dihydro-1H, 1'H-2,2'-biinden-1-ol
**6** (diastereomer) (*S,S and RR*) 2-Benzyl-2,3-dihydro-1H, 1'H-2,2'-biinden-1-ol
**7** (enantiomer) *(R,S or SR)* 2-Benzyl-2,3-dihydro-1H, 1'H-2,2'-biinden-1-ol
**8** (enantiomer) *(R,S or SR)* 2-Benzyl-2,3-dihydro-1H, 1'H-2,2'-biinden-1-ol
**9** (enantiomer) *(1R, 2R*) 2-Benzyl-2,3-dihydro-1H, 1'H-2,2'-biinden-1-ol
**10** (enantiomer) *(1S, 2S*) 2-Benzyl-2,3-dihydro-1H, 1'H-2,2'-biinden-1-ol

**13** (enantiomer) (*1S*,*28*)-2-benzyl-2,3-dihydro-2-(1H-inden-2-yl)-1H-inden-1-yl 4-bromobenzoate

APPENDIX 3:

**Table 3.** Atomic coordinates and equivalent isotropic atomic displacement parameters, (Å$^2$), for compound 13. U(eq) is defined as one third of the trace of the orthogonalised $U_{ij}$ tensor.

| | x/a | y/b | z/c | U(eq) |
|---|---|---|---|---|
| Br01 | 0.42895(10) | 0.0083(2) | 0.78773(8) | 0.0357(7) |
| O002 | 0.2578(7) | 0.6809(13) | 0.1477(7) | 0.025(2) |
| O003 | 0.2858(8) | 0.3367(14) | 0.1037(7) | 0.034(2) |
| C60 | 0.3732(10) | 0.8597(19) | 0.0418(9) | 0.024(3) |
| C005 | 0.3306(11) | 0.6500(19) | 0.0241(9) | 0.023(3) |
| C006 | 0.2916(10) | 0.537(2) | 0.0968(9) | 0.026(3) |
| C007 | 0.4041(11) | 0.966(2) | 0.9674(10) | 0.031(3) |
| C008 | 0.3943(9) | 0.853(2) | 0.8850(9) | 0.018(3) |
| C009 | 0.1880(10) | 0.678(2) | 0.3762(9) | 0.020(3) |
| C00A | 0.3266(10) | 0.542(2) | 0.9420(9) | 0.023(3) |
| C00B | 0.3562(11) | 0.644(2) | 0.8704(10) | 0.029(3) |
| C00C | 0.1335(10) | 0.476(2) | 0.4845(9) | 0.018(3) |
| C00D | 0.4693(10) | 0.015(3) | 0.3309(9) | 0.030(3) |
| C00E | 0.1729(11) | 0.671(2) | 0.5285(10) | 0.022(3) |
| C00F | 0.1292(9) | 0.558(2) | 0.6665(10) | 0.028(3) |
| C00G | 0.3952(11) | 0.8708(19) | 0.3368(9) | 0.024(3) |
| C00H | 0.0949(10) | 0.548(2) | 0.1620(9) | 0.026(3) |
| C00I | 0.1430(10) | 0.492(3) | 0.3881(8) | 0.025(3) |
| C00J | 0.2059(10) | 0.592(2) | 0.2139(9) | 0.024(3) |
| C00K | 0.2866(10) | 0.9439(18) | 0.3006(9) | 0.025(3) |
| C00L | 0.1705(11) | 0.715(2) | 0.6215(9) | 0.026(3) |
| C00M | 0.2092(11) | 0.811(2) | 0.4613(10) | 0.023(3) |
| C00N | 0.9493(12) | 0.372(3) | 0.0616(11) | 0.035(3) |
| C00O | 0.5703(12) | 0.970(2) | 0.3694(9) | 0.033(3) |
| C50 | 0.0915(11) | 0.318(2) | 0.5291(11) | 0.029(4) |
| C00Q | 0.5252(12) | 0.626(2) | 0.4254(9) | 0.028(3) |
| C00R | 0.2002(11) | 0.774(2) | 0.2853(10) | 0.024(3) |
| C00S | 0.0960(11) | 0.893(2) | 0.2378(10) | 0.027(3) |
| C00T | 0.0353(12) | 0.7170(19) | 0.1784(10) | 0.025(3) |
| C00U | 0.8894(11) | 0.538(3) | 0.0790(9) | 0.035(3) |
| C00V | 0.4201(11) | 0.672(2) | 0.3825(10) | 0.031(3) |
| C00W | 0.0887(10) | 0.359(2) | 0.6217(10) | 0.026(3) |
| C00X | 0.0547(12) | 0.376(2) | 0.1019(10) | 0.027(3) |
| C00Y | 0.9296(13) | 0.714(2) | 0.1355(11) | 0.035(3) |
| C00Z | 0.5975(12) | 0.772(2) | 0.4174(10) | 0.031(3) |

**Table 4.** Bond lengths (A) for compound **13**

| | | | |
|---|---|---|---|
| Br01-C008 | 1.891(12) | O002-C006 | 1.317(16) |
| O002-C00J | 1.465(16) | 0003-C006 | 1.227(17) |
| C60-C005 | 1.395(17) | C60-C007 | 1.440(18) |
| C005-C00A | 1.375(18) | C005-C006 | 1.494(18) |
| C007-C008 | 1.38(2) | C008-C00B | 1.366(17) |

(continued)

| | | | |
|---|---|---|---|
| C009-C00I | 1.318(19) | C009-C00M | 1.46(2) |
| C009-C00R | 1.522(18) | COOA-COOB | 1.385(19) |
| C00C-C50 | 1.373(19) | COOC-COOE | 1.390(19) |
| C00C-C00I | 1.478(16) | COOD-COOG | 1.36(2) |
| C00D-C00O | 1.37(2) | COOE-COOL | 1.418(19) |
| C00E-C00M | 1.496(19) | COOF-COOL | 1.371(19) |
| C00F-C00W | 1.416(19) | COOG-COOV | 1.382(18) |
| C00G-C00K | 1.503(19) | COOH-COOT | 1.371(18) |
| C00H-C00X | 1.388(19) | COOH-COOJ | 1.53(2) |
| C00J-C00R | 1.550(18) | COOK-COOR | 1.539(18) |
| C00N-C00U | 1.37(2) | COON-COOX | 1.40(2) |
| C00O-C00Z | 1.40(2) | C50-C00W | 1.41(2) |
| C00Q-C00Z | 1.35(2) | COOQ-COOV | 1.43(2) |
| C00R-C00S | 1.59(2) | COOS-COOT | 1.49(2) |
| C00T-C00Y | 1.41(2) | C00U-C00Y | 1.38(2) |

**Table 5.** Bond angles (°) for compound **13**

| | | | |
|---|---|---|---|
| C006-0002-C00J | 116.8(9) | C005-C60-C007 | 117.3(12) |
| C00A-C005-C60 | 121.3(13) | C00A-C005-C006 | 119.2(11) |
| C60-C005-C006 | 119.5(12) | 0003-C006-0002 | 124.6(12) |
| O003-C006-C005 | 124.0(12) | 0002-C006-C005 | 111.3(10) |
| C008-C007-C60 | 118.7(11) | C00B-C008-C007 | 123.0(13) |
| C00B-C008-Br01 | 120.3(11) | C007-C008-Br01 | 116.6(9) |
| C00I-C009-C00M | 110.3(12) | C00I-C009-C00R | 127.8(12) |
| C00M-C009-C00R | 120.9(11) | C005-C00A-C00B | 121.2(12) |
| C008-C00B-C00A | 118.1(14) | C50-C00C-C00E | 121.6(12) |
| C50-C00C-C00I | 132.4(13) | COOE-COOC-COOI | 106.0(11) |
| C00G-C00D-C00O | 121.9(14) | COOC-COOE-COOL | 120.9(12) |
| C00C-C00E-C00M | 109.0(12) | COOL-COOE-COOM | 130.0(12) |
| C00L-C00F-C00W | 121.5(12) | COOD-COOG-COOV | 120.4(13) |
| C00D-C00G-C00K | 117.9(11) | COOV-COOG-COOK | 121.5(12) |
| C00T-C00H-C00X | 121.7(13) | COOT-COOH-COOJ | 109.8(11) |
| C00X-C00H-C00J | 128.5(12) | C009-C00I-C00C | 110.8(12) |
| O002-C00J-C00H | 108.6(11) | O002-C00J-C00R | 108.7(9) |
| C00H-C00J-C00R | 104.1(10) | COOG-COOK-COOR | 119.9(10) |
| C00F-C00L-C00E | 117.7(12) | C009-C00M-C00E | 103.9(10) |
| C00U-C00N-C00X | 120.1(14) | COOD-COOO-COOZ | 118.9(14) |
| C00C-C50-C00W | 118.4(12) | COOZ-COOQ-COOV | 120.1(13) |
| C009-C00R-C00K | 113.0(11) | C009-C00R-C00J | 112.0(10) |
| C00K-C00R-C00J | 113.7(10) | C009-C00R-C00S | 105.7(11) |
| C00K-C00R-C00S | 108.7(10) | COOJ-COOR-COOS | 103.0(12) |
| C00T-C00S-C00R | 103.1(11) | COOH-COOT-COOY | 119.7(13) |
| C00H-C00T-C00S | 112.3(13) | COOY-COOT-COOS | 127.9(12) |
| C00N-C00U-C00Y | 121.9(14) | COOG-COOV-COOQ | 118.3(13) |
| C50-C00W-C00F | 120.0(13) | COOH-COOX-COON | 118.2(14) |
| C00U-C00Y-C00T | 118.3(14) | COOQ-COOZ-COOO | 120.5(14) |

**Table 6.** Anisotropic atomic displacement parameters ($Å^2$) for compound **13.** The anisotropic atomic displacement factor exponent takes the form: $-2\pi^2[ h^2 a^{*2} U_{11} + ... + 2 h k a^* b^* U_{12} ]$

| | $U_{11}$ | $U_{22}$ | $U_{33}$ | $U_{23}$ | $U_{13}$ | $U_{12}$ |
|---|---|---|---|---|---|---|
| Br01 1 | 0.0499(13) | 0.0487(9) | 0.0089(8) | 0.0045(7) | 0.0085(8) | -0.0069(8) |
| O002 | 0.034(6) | 0.028(4) | 0.017(5) | 0.003(4) | 0.014(5) | 0.000(4) |
| O003 | 0.045(6) | 0.036(4) | 0.024(6) | -0.003(4) | 0.018(6) | -0.002(5) |
| C60 | 0.038(9) | 0.034(6) | -0.001(7) | -0.001(6) | 0.000(7) | 0.001(6) |
| C005 | 0.034(9) | 0.027(5) | 0.007(7) | 0.003(5) | 0.006(7) | 0.009(6) |
| C006 | 0.035(9) | 0.032(6) | 0.013(7) | 0.005(6) | 0.007(7) | 0.007(7) |
| C007 | 0.041(9) | 0.030(6) | 0.021(7) | 0.007(5) | 0.010(8) | 0.003(6) |
| C008 | 0.013(8) | 0.040(6) | 0.000(6) | 0.012(5) | 0.001(6) | -0.003(6) |
| C009 | 0.016(9) | 0.039(6) | 0.003(6) | 0.008(5) | 0.000(6) | 0.008(6) |
| C00A | 0.025(8) | 0.036(7) | 0.006(6) | -0.005(6) | 0.000(6) | -0.007(7) |
| C00B | 0.043(10) | 0.030(5) | 0.011(7) | 0.009(5) | 0.002(8) | -0.006(6) |
| C00C | 0.018(8) | 0.030(6) | 0.001(6) | 0.004(5) | -0.006(6) | 0.008(6) |
| C00D | 0.038(6) | 0.033(5) | 0.026(7) | 0.000(8) | 0.024(7) | 0.004(7) |
| C00E | 0.022(9) | 0.038(6) | 0.007(6) | 0.003(5) | 0.003(7) | 0.001(6) |
| C00F | 0.019(8) | 0.055(8) | 0.006(7) | 0.003(6) | -0.003(7) | 0.001(6) |
| C00G | 0.034(6) | 0.038(6) | 0.003(7) | -0.011(6) | 0.010(7) | 0.000(5) |
| C00H | 0.039(6) | 0.039(6) | 0.002(6) | 0.001(5) | 0.010(6) | -0.006(5) |
| C00I | 0.028(8) | 0.041(6) | 0.004(6) | 0.010(6) | 0.000(7) | 0.007(7) |
| C00J | 0.039(7) | 0.037(5) | -0.005(6) | 0.004(5) | 0.001(6) | 0.001(5) |
| C00K | 0.038(6) | 0.030(6) | 0.010(7) | 0.003(5) | 0.012(7) | 0.000(4) |
| C00L | 0.030(9) | 0.045(7) | -0.006(6) | -0.006(6) | -0.012(7) | 0.000(6) |
| C00M | 0.026(9) | 0.038(6) | -0.003(6) | 0.009(5) | -0.013(7) | -0.004(6) |
| C00N | 0.040(8) | 0.060(8) | 0.008(8) | -0.005(7) | 0.008(7) | -0.010(7) |
| C00O | 0.037(7) | 0.053(7) | 0.012(7) | -0.005(7) | 0.011(7) | -0.015(7) |
| C50 | 0.037(10) | 0.033(6) | 0.024(8) | -0.002(6) | 0.019(9) | -0.002(6) |
| C00Q | 0.041(7) | 0.042(6) | 0.000(7) | -0.002(6) | 0.003(7) | -0.002(6) |
| C00R | 0.030(6) | 0.038(6) | 0.009(6) | -0.001(5) | 0.014(6) | 0.000(5) |
| C00S | 0.038(7) | 0.031(5) | 0.009(6) | 0.006(5) | -0.002(6) | 0.001(5) |
| C00T | 0.037(7) | 0.036(6) | 0.002(7) | 0.006(5) | 0.003(6) | -0.002(6) |
| C00U | 0.029(8) | 0.067(9) | 0.008(7) | 0.000(7) | 0.003(7) | -0.004(6) |
| C00V | 0.034(7) | 0.047(6) | 0.011(8) | 0.006(6) | 0.007(7) | -0.006(6) |
| C00W | 0.019(9) | 0.045(7) | 0.004(7) | 0.008(6) | -0.010(7) | 0.007(6) |
| C00X | 0.038(7) | 0.035(6) | 0.007(8) | 0.004(6) | 0.001(7) | 0.001(6) |
| C00Y | 0.036(7) | 0.051(7) | 0.012(8) | 0.006(6) | -0.004(7) | -0.004(7) |
| C00Z | 0.029(8) | 0.056(7) | 0.005(8) | -0.019(6) | 0.000(7) | 0.005(6) |

**Table 7.** Hydrogen atomic coordinates and isotropic atomic displacement parameters ($Å^2$) for compound **13**

| | x/a | y/b | z/c | U(eq) |
|---|---|---|---|---|
| H60 | 0.3814 | 0.9294 | 0.1005 | 0.029 |
| H007 | 0.4307 | 1.1117 | -0.0251 | 0.037 |
| H00A | 0.3030 | 0.3937 | -0.0657 | 0.028 |
| H00B | 0.3502 | 0.5708 | -0.1873 | 0.035 |
| H00D | 0.4506 | 1.1511 | 0.2992 | 0.035 |
| H00F | 0.1278 | 0.5827 | 0.7292 | 0.034 |
| H00I | 0.1197 | 0.3839 | 0.3410 | 0.03 |
| H00J | 0.2401 | 0.4558 | 0.2454 | 0.029 |
| H00C | 0.2802 | 1.0186 | 0.2401 | 0.03 |

(continued)

| | x/a | y/b | z/c | U(eq) |
|---|---|---|---|---|
| H00E | 0.2740 | 1.0566 | 0.3442 | 0.03 |
| H00L | 0.1967 | 0.8488 | 0.6516 | 0.031 |
| H53 | 0.1717 | 0.9523 | 0.4499 | 0.028 |
| H51 | 0.2828 | 0.8419 | 0.4851 | 0.028 |
| H00N | -0.0805 | 0.2526 | 0.0222 | 0.043 |
| H00O | 0.6210 | 1.0723 | 0.3636 | 0.04 |
| H00P | 0.0650 | 0.1854 | 0.4983 | 0.035 |
| H00Q | 0.5442 | 0.4932 | 0.4594 | 0.033 |
| H52 | 0.0625 | 0.9454 | 0.2851 | 0.033 |
| H50 | 0.1064 | 1.0200 | 0.1995 | 0.033 |
| H00U | -0.1818 | 0.5319 | 0.0514 | 0.042 |
| H00V | 0.3689 | 0.5684 | 0.3854 | 0.037 |
| H00W | 0.0596 | 0.2536 | 0.6540 | 0.031 |
| H00X | 0.0973 | 0.2645 | 0.0885 | 0.033 |
| H00Y | -0.1127 | 0.8309 | 0.1453 | 0.042 |
| H00Z | 0.6671 | 0.7386 | 0.4446 | 0.037 |

**Claims**

1. A compound of the absolute stereochemical formula below and pharmaceutically acceptable salts thereof

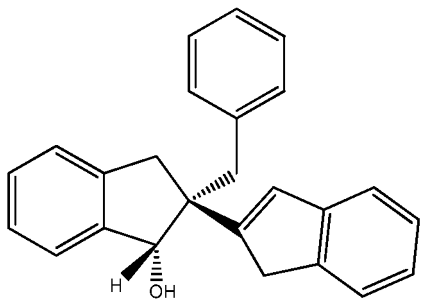

2. A pharmaceutical composition comprising an effective amount of a compound as claimed in claim 1 and a pharmaceutically acceptable carrier.

3. A compound as claimed in claim 1 for use as a medicament in the prophylaxis or treatment of an autoimmune disease which involves T-cell proliferation or function.

4. A compound as claimed in claim 1 for use as a medicament intended for prophylaxis or treatment of inflammatory bowel disease.

5. A compound as claimed in claim 1 for use as a medicament intended for prophylaxis or treatment of ulcerative colitis.

6. A compound as claimed in claim 1 for use as a medicament intended for prophylaxis or treatment of Crohn’s disease.

**Patentansprüche**

1. Verbindung der nachstehenden absoluten stereochemischen Formel und pharmazeutisch verträgliche Salze davon:

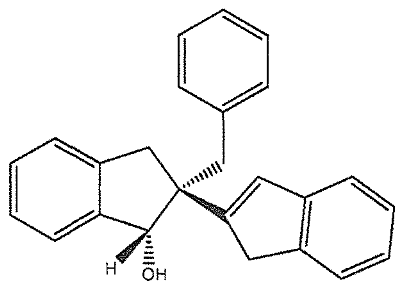

.

2. Pharmazeutische Zusammensetzung, die eine wirksame Menge einer Verbindung nach Anspruch 1 und einen pharmazeutisch verträglichen Träger umfasst.

3. Verbindung nach Anspruch 1 für die Verwendung als Medikament bei der Prophylaxe oder Behandlung einer Autoimmunerkrankung, die die T-Zell-Proliferation oder -Funktion involviert.

4. Verbindung nach Anspruch 1 für die Verwendung als Medikament, das zur Prophylaxe oder Behandlung von entzündlicher Darmerkrankung vorgesehen ist.

5. Verbindung nach Anspruch 1 für die Verwendung als Medikament, das zur Prophylaxe oder Behandlung von Colitis ulcerosa vorgesehen ist.

6. Verbindung nach Anspruch 1 für die Verwendung als Medikament, das zur Prophylaxe oder Behandlung von Morbus Crohn vorgesehen ist.

**Revendications**

1. Composé répondant à la formule stéréochimique absolue ci-après et les sels pharmaceutiquement acceptables de celui-ci

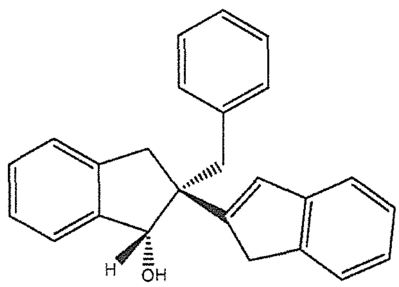

2. Composition pharmaceutique, comprenant une quantité efficace d'un composé selon la revendication 1 et un véhicule pharmaceutiquement acceptable.

3. Composé selon la revendication 1, pour une utilisation comme médicament destiné à la prophylaxie ou au traitement d'une maladie auto-immune qui implique une fonction ou prolifération des lymphocytes T.

4. Composé selon la revendication 1, pour une utilisation comme médicament destiné à la prophylaxie ou au traitement de l'affection abdominale inflammatoire.

5. Composé selon la revendication 1, pour une utilisation comme médicament destiné à la prophylaxie ou au traitement de la rectocolite hémorragique.

6. Composé selon la revendication 1, pour une utilisation comme médicament destiné à la prophylaxie ou au traitement de la maladie de Crohn.

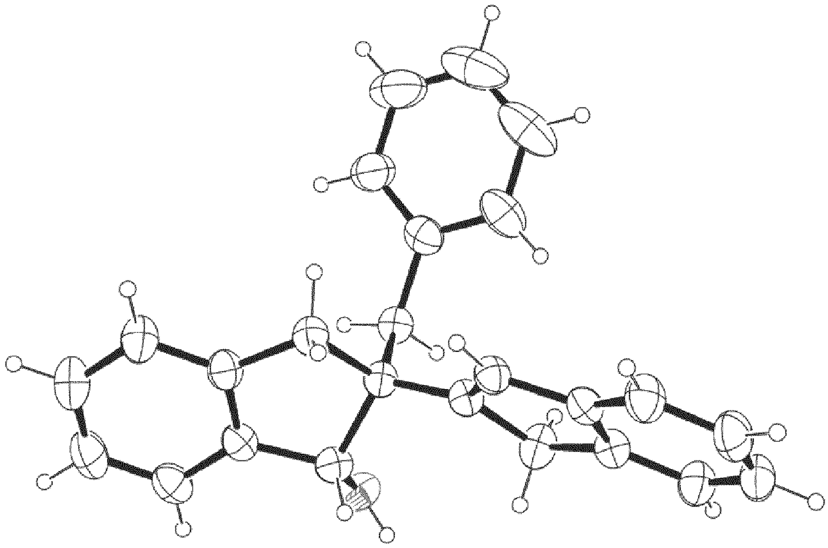

Fig. 1

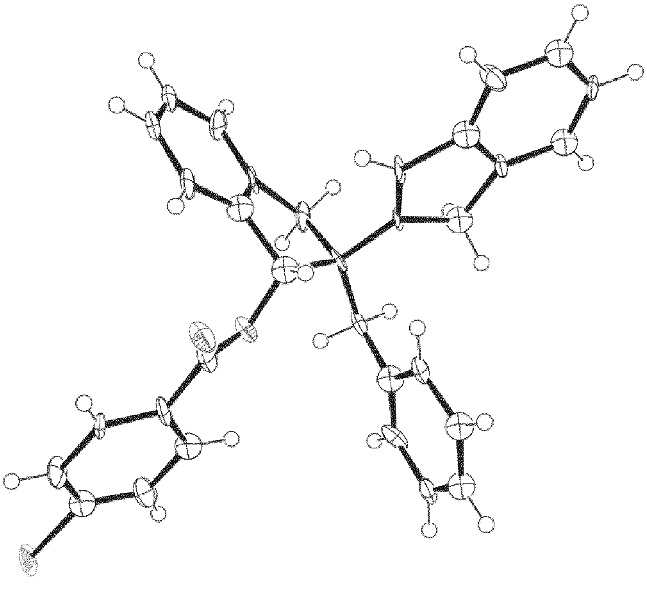

Fig. 2

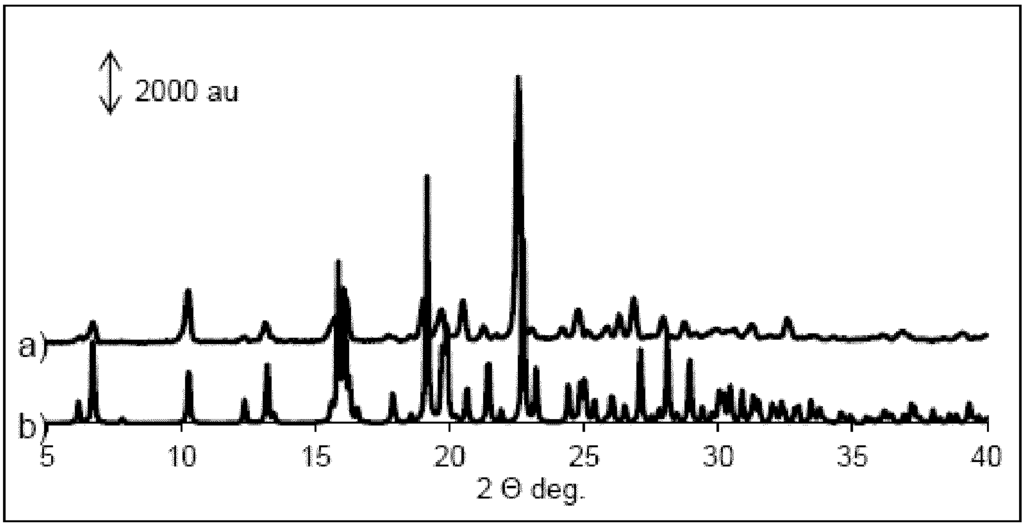
2000 au
a)
b)
5
10
15
20
25
30
35
40
2 Θ deg.

**Fig. 3**

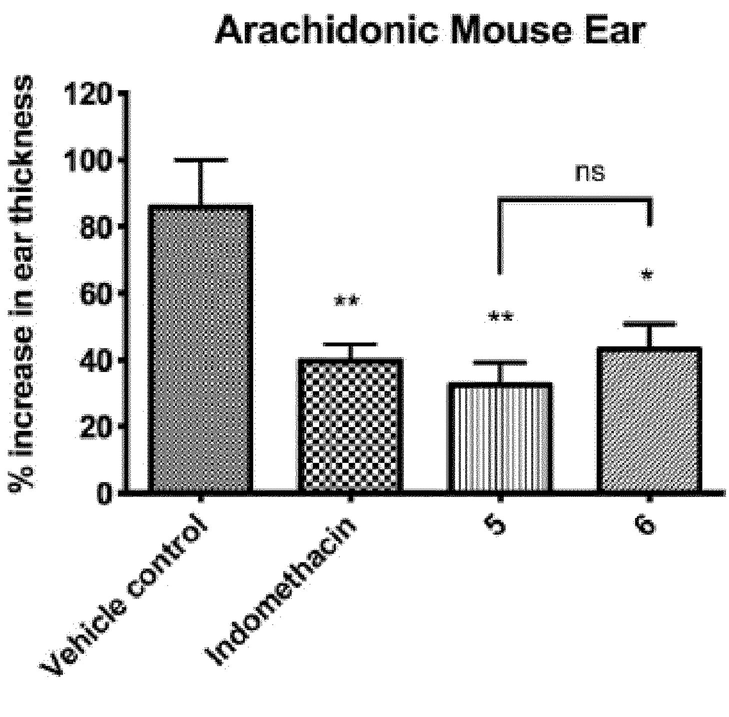
Arachidonic Mouse Ear
% increase in ear thickness
120
100
80
60
40
20
0
ns
**
**
*
Vehicle control
Indomethacin
5
6

**Fig. 4**

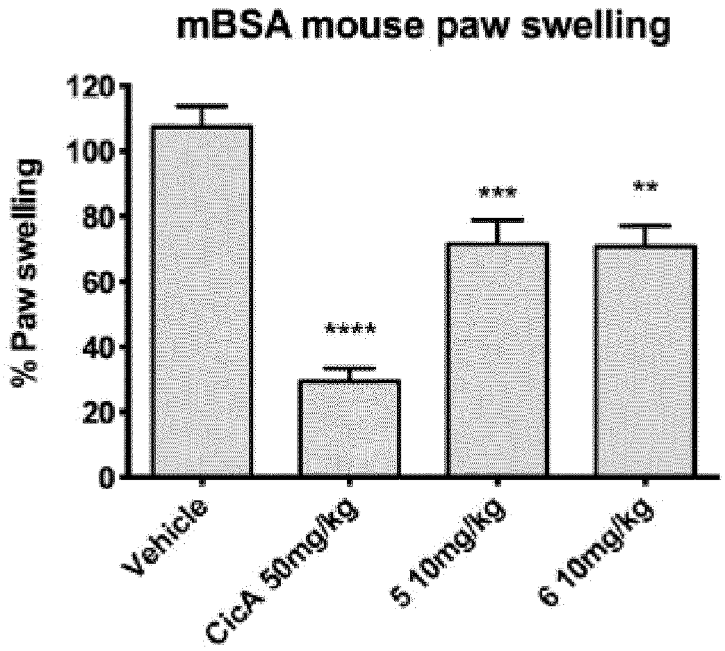
mBSA mouse paw swelling
% Paw swelling
120
100
80
60
40
20
0
****
***
**
Vehicle
CicA 50mg/kg
5 10mg/kg
6 10mg/kg

Fig. 5

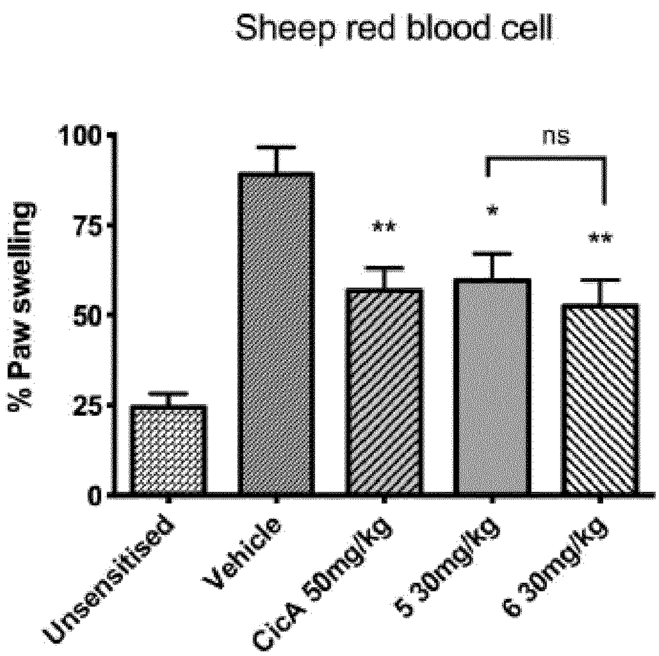
Sheep red blood cell
% Paw swelling
100
75
50
25
0
ns
**
*
**
Unsensitised
Vehicle
CicA 50mg/kg
5 30mg/kg
6 30mg/kg

Fig. 6

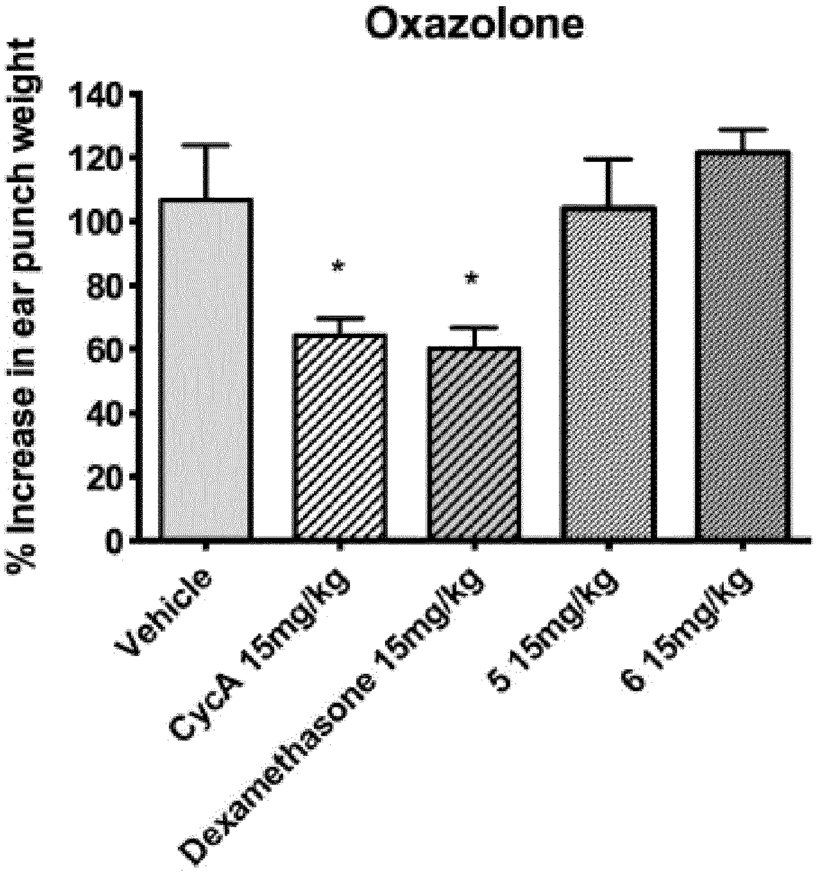
Oxazolone
% Increase in ear punch weight
140
120
100
80
60
40
20
0
*
*
Vehicle
CycA 15mg/kg
Dexamethasone 15mg/kg
5 15mg/kg
6 15mg/kg

Fig. 7

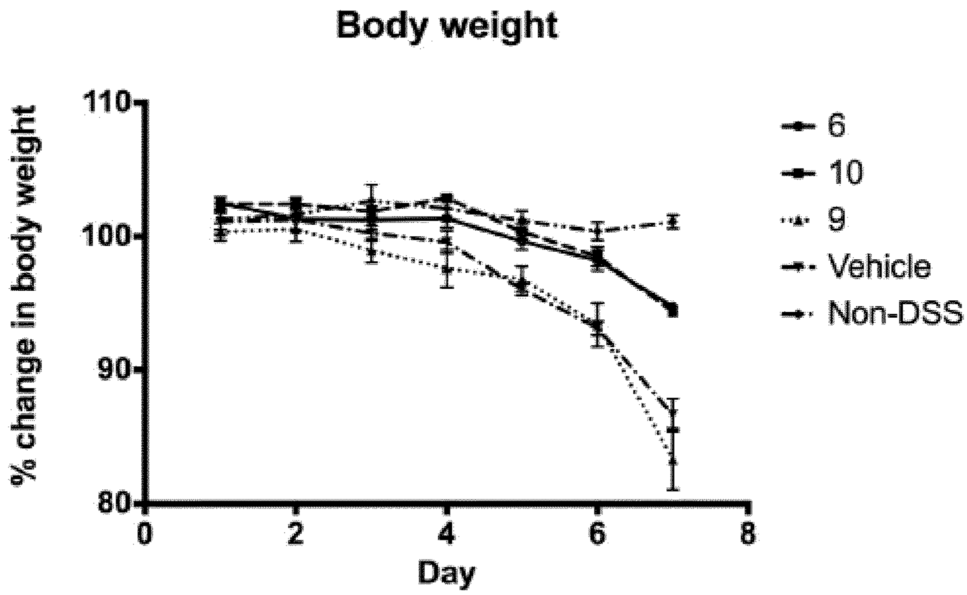
Body weight
110
100
90
80
% change in body weight
0
2
4
6
8
Day
6
10
9
Vehicle
Non-DSS

Fig. 8

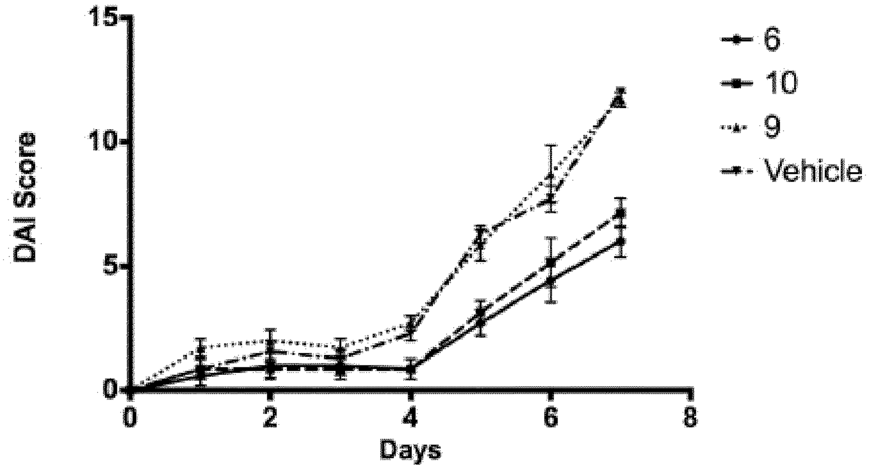
15
10
5
0
DAI Score
0
2
4
6
8
Days
6
10
9
Vehicle

Fig. 9

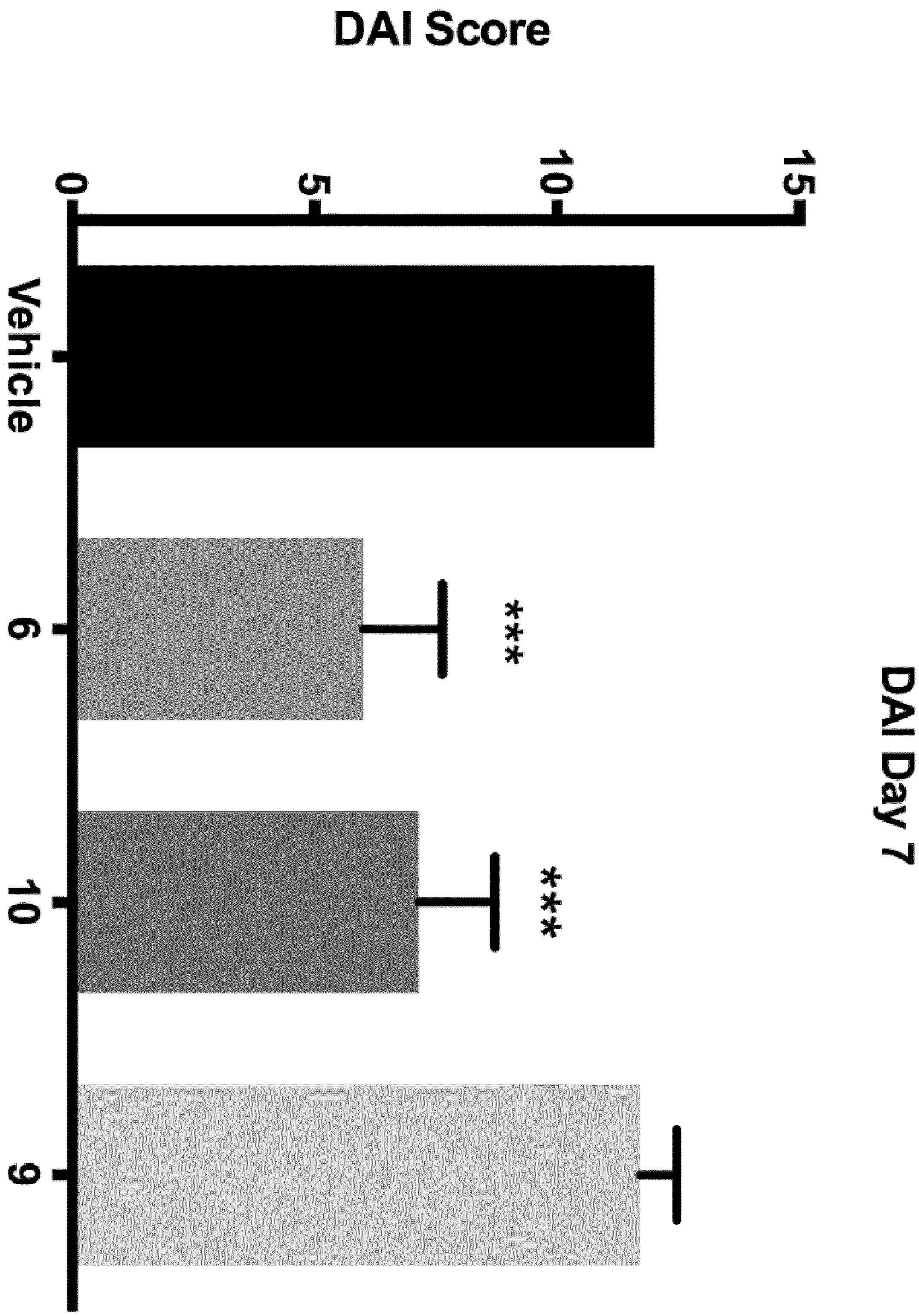


Fig. 10

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 9720802 A


### Non-patent literature cited in the description


- *Inflamm. Res.,* 2008, vol. 57, 15
- *Bioorganic & Medicinal Chemistry Letters,* 2009, vol. 19 (20), 5927-5930
- *Bioorganic & Medicinal Chemistry Letters,* 2009, vol. 15;19 (20), 5927-30
- **SHOSHANA D. KATZMAN ; KATRINA K. HOYER ; HANS DOOMS ; IRIS K. GRATZ ; MICHAEL D. ROSENBLUM ; JONATHAN S. PAW ; SARA H. ISAKSON ; ABUL K. ABBAS.** *Cytokine,* 2011, vol. 56, 116-121
- *Cytokine & Growth Factor Reviews,* 2002, vol. 13, 369-378
- *Cell,* 1993, vol. 75, 253-61